# EUROPEAN PATENT APPLICATION

(11) **EP 3 628 737 A1**
(43) Date of publication of application: **01.04.2020**
(21) Application number: 18827723.0
(22) Date of filing: 03.07.2018
(51) Int. Cl.: C12N 15/113, A61K 31/712, A61P 25/16, A61P 25/28, A61P 43/00

(54) **ENA ANTISENSE OLIGONUCLEOTIDE FOR INHIBITION OF ALPHA-SYNUCLEIN EXPRESSION**

(30) Priority: 05.07.2017 JP 2017132290
(71) Applicant: Osaka University, Suita-shi, Osaka 565-0871 (JP); Daiichi Sankyo Company, Limited, Tokyo 103-8426 (JP)
(72) Inventor: NAKAMORI, Masayuki, Suita-shi Osaka 565-0871 (JP); MOCHIZUKI, Hideki, Suita-shi Osaka 565-0871 (JP); OBIKA, Satoshi, Suita-shi Osaka 565-0871 (JP); KOIZUMI, Makoto, Tokyo 103-8426 (JP); NAKAMURA, Akifumi, Tokyo 103-8426 (JP); TAKAISHI, Kiyosumi, Tokyo 103-8426 (JP); ASAHI, Yumiko, Tokyo 103-8426 (JP)
(74) Representative: Schlich, George
(86) International application number: PCT/JP2018/025237
(87) International publication number: WO 2019/009299

(57) **Abstract**

The objective of the present invention is to provide nucleic acid therapeutics which exhibits more excellent effect and which shows a substantivity for a prolonged period to suppress an expression of α-synuclein. The oligonucleotide or a pharmacologically acceptable salt thereof according to the present invention is characterized in comprising at least one 2'-0,4'-C-ethylene nucleoside, wherein the oligonucleotide can hybridize with α-synuclein gene, has an activity to suppress an expression of the α-synuclein gene, and is complementary to the α-synuclein gene, 5' end of the oligonucleotide is a nucleotide complementary to the specific nucleotide, the oligonucleotide is complementary to at least a part of SEQ ID NO: 1, and the oligonucleotide has a length of 13 or more and 15 or less nucleotides.

## Description

### TECHNICAL FIELD

The present invention relates to a novel oligonucleotide having an α-synuclein expression suppressing action, an α-synuclein expression inhibitor containing the novel oligonucleotide, and in more detail an α-synuclein expression inhibitor utilizing a novel artificial nucleic acid.

### BACKGROUND ART

Parkinson's disease (PD) can be classified into sporadic Parkinson's disease and hereditary Parkinson's disease.

Sporadic Parkinson's disease is a progressive neurodegenerative disease, and the prevalence rate thereof is one in one thousand people. When the disease progresses, dementia is combined. Such dementia is Lewy body dementia, and there are supportive measures only for treating the dementia. Sporadic Parkinson's disease is considered to be caused by the aggregation and accumulation of α-synuclein in the brain.

Hereditary Parkinson's disease accounts for 5 to 10% of Parkinson's disease, and PARK4 gene among pathogenic genes PARK1 to PARK20 is considered to involve the disease. Hereditary Parkinson's disease caused by PARK4 gene is autosomal-dominantly inherited. There are dozens of hereditary Parkinson's disease patients in Japan. In hereditary Parkinson's disease caused by PARK4 gene, normal α-synuclein gene is excessively expressed and parkinsonian symptom is combined with dementia.

α-Synuclein is a protein composed of 140 amino acid residues and is an amyloid protein which does not have a specific native structure. α-Synuclein involves the accumulation and release of synaptic vesicle. An α-synuclein knockout (KO) mouse pathologically reveals no abnormality and can exhibit neuroprotective action against neurotoxic MPTP (1-methyl-4-phenyl-1,2,3,6-tetrahydropyridine) .

α-Synuclein is a main component of Lewy body in a disease such as Parkinson's disease and Lewy body dementia (DLB). When a relationship between disease progression and α-synuclein lesion was assessed by staining α-synuclein in an autopsy brain for a stage classification of PD autopsy brain analysis Braak, it was found that an aggregation of α-synuclein in a neuron was a main part of the lesion. In addition, when α-synuclein fibril was administered to an α-synuclein transgenic (Tg) mouse, the lesion was extended from the fibril as a core and abnormal α-synuclein was also observed out of a cell. This phenomenon is referred to as prion-like extracellular propagation.

A clinical condition of Parkinson's disease is hereinafter described. Mesaticephalic black nerve cells are denatured and a production amount of dopamine is decreased by an aggregation of abnormal α-synuclein in a neuron. As a result, motility disturbance or cognitive disorder is caused. In a conventional symptomatic therapy, a nerve degeneration gradually progresses, and an L-dopa formulation is administered for assorting dopamine or a dopamine agonist is administered for stimulating dopamine secretion against a decrease of dopamine production.

Nucleic acid therapeutics for knockdown of α-synuclein has been tried to be used to target an aggregation of abnormal α-synuclein in a neuron.

With respect to nucleic acid therapeutics for suppressing excess α-synuclein, use of adeno associated virus (AAV) ribozyme in a rat (Non-patent document 1), use of lentivirus-shRNA in a rat (Non-patent document 2), use of AAV-shRNA in a rat (Non-patent documents 3 and 4), use of naked siRNA in a mouse (Non-patent document 5), use of exosome siRNA in a mouse (Non-patent document 6), and use of siRNA (2-O-Me) in a monkey (Non-patent document 7) are reported. There are however problems that virus is used in Non-patent documents 1 to 4, the effect of siRNA described in Non-patent documents 5 and 6 is immediately lost, and the effect of siRNA described in Non-patent document 7 is insufficient.

It is reported to use an artificial nucleic acid to suppress an expression of α-synuclein gene (Patent document 1). A nucleoside modified by 2'-O-methoxyethyl (MOE) is used in Patent document 1. In addition, an oligonucleotide is administered by injection through an intrastriatal bolus injection in Patent document 1.

### PRIOR ART DOCUMENT

### PATENT DOCUMENT

Patent document 1: JP 2014-501507 T

### NON-PATENT DOCUMENT

Non-patent document 1: Kinoh et al., BBRC, 2006, vol. 341, pp. 1088-95
Non-patent document 2: Sapru et al., ExpNeurol, 2006, vol. 198, pp. 382-90
Non-patent document 3: Gorbatyuk et al., MolTher, 2010, vol. 18, pp. 1450-7
Non-patent document 4: Khodr et al., Brain Res, 2011, vol. 1395, pp. 94-107
Non-patent document 5: Lewis et al., MolNeurodegener, 2008, vol. 3, pp. 19
Non-patent document 6: Cooper et al., MovDisord, 2014, vol. 29, pp. 1476-85
Non-patent document 7: McCormack et al., PLoS One, 2010, vol. 5, pp. e12122

### DISCLOSURE OF THE INVENTION

### PROBLEMS TO BE SOLVED BY THE INVENTION

The above-described problems can be solved by the present invention, and the objective of the present invention is to provide nucleic acid therapeutics which exhibits more excellent effect and which shows a substantivity for a prolonged period to suppress an expression of α-synuclein.

### MEANS FOR SOLVING THE PROBLEMS

The present invention provides an oligonucleotide or a pharmacologically acceptable salt thereof,
comprising at least one 2'-O,4'-C-ethylene nucleoside,
wherein the oligonucleotide can hybridize with α-synuclein gene, has an activity to suppress an expression of the α-synuclein gene, and is complementary to the α-synuclein gene,
5' end of the oligonucleotide is a nucleotide complementary to any one nucleotide selected from the group consisting of the 40^{th} to 43^{rd} positions, the 74^{th} to 76^{th} positions, the 215^{th} position, the 227^{th} to 230^{th} positions, the 234^{th} position, the 254^{th} position, the 255^{th} position, the 263^{rd} position, the 266^{th} to 269^{th} positions, the 273^{rd} to 275^{th} positions, the 277^{th} position, the 278^{th} position, the 284^{th} to 286^{th} positions, the 288^{th} position, the 289^{th} position, the 366^{th} to 368^{th} positions, and the 412^{nd} to 415^{th} positions of SEQ ID NO: 1,
the oligonucleotide is complementary to at least a part of SEQ ID NO: 1, and
the oligonucleotide has a length of 13 or more and 16 or less nucleotides.

As one embodiment, the 5' end of the oligonucleotide or pharmacologically acceptable salt thereof is a nucleotide complementary to any one nucleotide selected from the group consisting of the 40^{th} to 42^{nd} positions, the 74^{th} to 76^{th} positions, the 215^{th} position, the 227^{th} to 230^{th} positions, the 234^{th} position, the 254^{th} position, the 255^{th} position, the 263^{rd} position, the 266^{th} position, the 267^{th} position, the 269^{th} position, the 273^{rd} to 275^{th} positions, the 277^{th} position, the 278^{th} position, the 284^{th} to 286^{th} positions, the 288^{th} position, the 289^{th} position, the 366^{th} to 368^{th} positions, and the 412^{nd} to 415^{th} positions of SEQ ID NO: 1, the oligonucleotide or pharmacologically acceptable salt thereof is complementary to at least a part of SEQ ID NO: 1, and has a length of 13 or more and 16 or less nucleotides.

As one embodiment, the 5' end of the oligonucleotide or pharmacologically acceptable salt thereof is a nucleotide complementary to any one nucleotide selected from the group consisting of the 41^{st} position, the 42^{nd} position, the 215^{th} position, the 227^{th} to 230^{th} positions, the 234^{th} position, the 274^{th} position, the 277^{th} position, the 278^{th} position, the 284^{th} to 286^{th} positions, the 288^{th} position, the 366^{th} to 368^{th} positions, and the 412^{nd} to 414^{th} positions of SEQ ID NO: 1, the oligonucleotide or pharmacologically acceptable salt thereof is complementary to at least a part of SEQ ID NO: 1, and has a length of 13 or more and 16 or less nucleotides.

As one embodiment, the 5' end of the oligonucleotide or pharmacologically acceptable salt thereof is a nucleotide complementary to any one nucleotide selected from the group consisting of the 42^{nd} position, the 227^{th} to 230^{th} positions, the 274^{th} position, the 277^{th} position, the 278^{th} position, the 284^{th} to 286^{th} positions, the 413^{rd} position, and the 414^{th} position of SEQ ID NO: 1, the oligonucleotide or pharmacologically acceptable salt thereof is complementary to at least a part of SEQ ID NO: 1, and has a length of 13 or more and 16 or less nucleotides.

As one embodiment, the 5' end of the oligonucleotide or pharmacologically acceptable salt thereof is a nucleotide complementary to any one nucleotide selected from the group consisting of the 42^{nd} position, the 227^{th} position, the 229^{th} position, the 274^{th} position, the 277^{th} position, the 278^{th} position, the 285^{th} position, and the 413^{rd} position of SEQ ID NO: 1, the oligonucleotide or pharmacologically acceptable salt thereof is complementary to at least a part of SEQ ID NO: 1, and has a length of 13 or more and 16 or less nucleotides.

As one embodiment, the 5' end of the oligonucleotide or pharmacologically acceptable salt thereof is a nucleotide complementary to any one nucleotide selected from the group consisting of the 227^{th} position, the 229^{th} position, the 278^{th} position, the 285^{th} position, and the 413^{rd} position of SEQ ID NO: 1, the oligonucleotide or pharmacologically acceptable salt thereof is complementary to at least a part of SEQ ID NO: 1, and has a length of 13 or more and 16 or less nucleotides.

As one embodiment, the oligonucleotide or pharmacologically acceptable salt has the 5' end complementary to any one nucleotide selected from the group consisting of the 229^{th} position, the 278^{th} position, the 285^{th} position and the 413^{rd} position of SEQ ID NO: 1, is complementary to at least a part of SEQ ID NO: 1, and has a length of 15 nucleotides; or the oligonucleotide or pharmacologically acceptable salt has the 5' end complementary to the 227^{th} position of SEQ ID NO: 1, is complementary to at least a part of SEQ ID NO: 1, and has a length of 13 nucleotides.

As a further embodiment, the oligonucleotide is a gapmer consisting of a gap region having a length of 5 or more and 7 or less bases, a 5' wing having a length of 3 or more and 5 or less bases, and a 3' wing having a length of 3 or more and 5 or less bases,
the gap region is placed between the 5' wing and the 3' wing, and
the 5' wing and the 3' wing comprise at least one 2'-O,4'-C-ethylene nucleoside.

In this disclosure, 2'-O,4'-C-ethylenenucleoside is described as ENA (2'-O,4'-C-Ethylene-bridged Nucleic Acid) in some cases.

The 5' wing and the 3' wing may contain a nucleoside modified to be a 2'-O-alkylated nucleoside, or by AmNA or S-cEt (2',4'-constrained ethyl) described in a document (Yahara, A. et al., ChemBioChem (2012), 13, 2513-2516) or WO 2014/109384.

As a 2'-O-alkylated nucleoside, a 2'-O-alkylated nucleoside of D-ribofuranose may be used. An example of 2'-O-alkylated includes 2'-O-methylated, 2'-O-aminoethylated, 2'-O-propylated, 2'-O-allylated, 2'-O-methoxyethylated, 2'-O-butylated, 2'-O-pentylated and 2'-O-propargylated.

Also, the resent invention provides an α-synuclein expression inhibitor comprising the above-described oligonucleotide or pharmacologically acceptable salt thereof as an active ingredient.

Further, the present invention provides a pharmaceutical composition comprising the above-described oligonucleotide or pharmacologically acceptable salt thereof as an active ingredient.

As one embodiment, the pharmaceutical composition is used for treating or preventing α-synuclein excess symptom.

As one embodiment, the pharmaceutical composition is used for treating or preventing Parkinson's disease or Lewy body dementia.

Further, the present invention provides a method for suppressing an expression of α-synuclein, comprising the step of administering the above-described oligonucleotide or pharmacologically acceptable salt thereof to a subject.

Further, the present invention provides a method for treating or preventing α-synuclein excess symptom, comprising the step of administering the above-described oligonucleotide or pharmacologically acceptable salt thereof to a subject.

Further, the present invention provides a method for treating or preventing Parkinson's disease or Lewy body dementia, comprising the step of administering the above-described oligonucleotide or pharmacologically acceptable salt thereof to a subject.

### EFFECT OF THE INVENTION

The present invention provides an oligonucleotide having the effect to suppress an expression of α-synuclein and a substantivity. The effect to suppress an expression of α-synuclein by the oligonucleotide can be also exhibited by intrathecal administration, which is a general administration route used for a clinical application, according to the present invention.

### BRIEF DESCRIPTION OF THE DRAWINGS

Figure 1 is a graph to demonstrate the amount of α-synuclein mRNA after the antisense oligonucleotide (ASO) was transfected into HEK293A cell in Test example 1.
Figure 2 is a graph to demonstrate the amount of α-synuclein mRNA after the antisense oligonucleotide (ASO) was transfected into HEK293A cell in Test example 2.
Figure 3 is a graph to demonstrate the amount of α-synuclein mRNA after the antisense oligonucleotide (ASO) was transfected into HEK293A cell in Test example 3.
Figure 4 is a graph to demonstrate the amount of α-synuclein mRNA after the antisense oligonucleotide (ASO) was transfected into HEK293A cell in Test example 4.
Figure 5 is a graph to demonstrate the amount of α-synuclein mRNA after the antisense oligonucleotide (ASO) was transfected into HEK293A cell in Test example 5.

### MODE FOR CARRYING OUT THE INVENTION

First, the terms used in this disclosure are defined.

In this disclosure, the term "nucleoside" means a "nucleoside" in which a purine base or a pyrimidine base is bound to a sugar. A naturally occurring type nucleoside is referred to as a "natural nucleoside" in some cases. A modified non-natural nucleoside is referred to as a "modified nucleoside" in some cases. In particular, a nucleotide of which sugar part is modified is referred to as a "sugar-modified nucleoside". The term "nucleotide" means a compound in which a phosphate group is bound to a sugar of a nucleoside.

In this disclosure, the term "oligonucleotide" means a polymer of "nucleotide" and is formed by binding 2 or more and 50 or less of the same or different nucleosides through a phosphodiester bond or other bond. There are a natural oligonucleotide and a non-natural oligonucleotide in the oligonucleotide. An example of a non-natural oligonucleotide preferably includes a sugar derivative of which sugar part is modified; a phosphorothioate derivative formed by replacing a non-crosslinking oxygen atom of a phosphodiester bond with a sulfur atom; an ester derivative formed by esterifying a phosphodiester bond; and an amide derivative formed by amidating an amino group on a purine base, and more preferably includes a sugar derivative of which sugar part is modified; a phosphorothioate derivative formed by replacing a non-crosslinking oxygen atom of a phosphodiester bond with a sulfur atom; or a derivative containing both of a "sugar derivative of which sugar part is modified" and a "phosphorothioate formed by replacing a non-crosslinking oxygen atom of a phosphodiester bond by a sulfur atom".

In this disclosure, the term "antisense oligonucleotide" is described as AON or ASO, means an oligonucleotide complementary to an mRNA, an mRNA precursor or an ncRNA (noncoding RNA) of a target gene, and is composed of a single-stranded DNA, RNA and/or analogue thereof. When the antisense oligonucleotide forms a duplex with a target mRNA, mRNA precursor or ncRNA, a function of the mRNA, mRNA precursor or ncRNA is suppressed. The "antisense oligonucleotide" may be completely complementary to a target mRNA, mRNA precursor or ncRNA. Alternatively, the "antisense oligonucleotide" may contain one or several mismatches or a base which forms a wobble base pair as long as the antisense oligonucleotide can hybridize with a target mRNA, mRNA precursor or ncRNA and can suppress a function of the mRNA, mRNA precursor or ncRNA. An analogue of DNA or RNA means a molecule having a structure similar to that of the DNA or RNA. An example of such an analogue includes a peptide nucleic acid (PNA). An ncRNA (noncoding RNA) is a generic term of RNA which functions without being translated to a protein. An example of an ncRNA includes ribosome RNA, transfer RNA and miRNA.

In this disclosure, the term "pharmacologically acceptable salt" means a salt of the oligonucleotide according to the present invention, and a physiologically acceptable and pharmaceutically acceptable salt of the oligonucleotide according to the present invention, in other words, a salt which retains a desired biological activity of the oligonucleotide and which does not exhibit an undesired toxicological effect. An example of such a salt includes an alkali metal salt such as sodium salt, potassium salt and lithium salt; an alkaline earth metal salt such as calcium salt and magnesium salt; a metal salt such as aluminum salt, iron salt, zinc salt, copper salt, nickel salt and cobalt salt; an inorganic salt such as ammonium salt; an amine salt such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt and tris(hydroxymethyl)aminomethane salt; a hydrohalic acid salt such as hydrofluoride salt, hydrochloride salt, hydrobromide salt and hydroiodide salt; an inorganic acid salt such as nitrate salt, perchlorate salt, sulfate salt and phosphate salt; a lower alkane sulfonate salt such as methanesulfonate salt, trifluoromethanesulfonate salt and ethanesulfonate salt; an aryl sulfonate salt such as benzenesulfonate salt and p-toluenesulfonate salt; an organic acid salt such as acetate salt, malate salt, fumarate salt, succinate salt, citrate salt, tartrate salt, oxalate salt and maleate salt; and an amino acid salt such as glycine salt, lysine salt, arginine salt, ornithine salt, glutamate salt and aspartate salt.

Hereinafter, the present invention is described in detail.

The oligonucleotide of the present invention may be an oligonucleotide of which natural DNA or RNA is chemically modified. Such a modification has an affect on the activity of the oligonucleotide. For example, the modification increases an affinity for a target gene and a resistance to a nucleic acid degrading enzyme (nuclease), and has an affect on a pharmacokinetics and a tissue distribution of the oligonucleotide. It may become possible to use the shorter oligonucleotide by improving an affinity of the oligonucleotide for a target.

The present invention relates to the oligonucleotide and pharmacologically acceptable salt thereof as hereinafter described.

The oligonucleotide of the present invention contains at least one 2'-O,4'-C-ethylene nucleoside at an arbitrary position. The 2'-O,4'-C-ethylene nucleoside has an ethylene bridge between the 2^{nd} position and the 4^{th} position of the sugar ring.

The base part of the oligonucleotide is preferably a group represented by the following structural formulae: 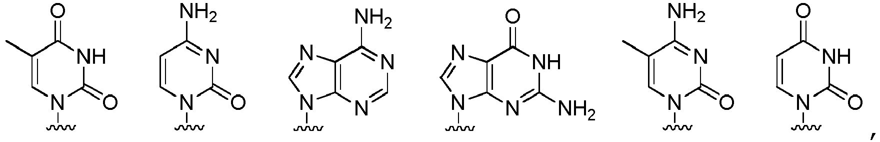 specifically thyminyl group, cytosinyl group, adeninyl group, guaninyl group, 5-methylcytosinyl group and uracilyl group, and 2-oxo-4-hydroxy-5-methyl-1,2-dihydropyrimidine-1-yl group, 2-oxo-4-amino-1,2-dihydropyrimidine-1-yl group, 6-aminopurine-9-yl group, 2-amino-6-hydroxypurine-9-yl group, 4-amino-5-methyl-2-oxo-1,2-dihydropyrimidine-1-yl group and 2-oxo-4-hydroxy-1,2-dihydropyrimidine-1-yl group, and particularly preferably thyminyl group, cytosinyl group, adeninyl group, guaninyl group, 5-methylcytosinyl group and uracilyl group. When the oligonucleotide is synthesized, the hydroxyl group and amino group are preferably protected with a protective group. Uracil (U) and thymine (T) are compatible among nucleobases. Both of uracil (U) and thymine (T) can be used for forming a base pair with adenine (A) in a complementary strand.

For example, the above-described oligonucleotide containing at least one 2'-O,4'-C-ethylene nucleoside can be synthesized by the method described in WO 2000/47599 with using 2'-O,4'-C-ethylene nucleoside phosphoramidite.

The oligonucleotide of the present invention can hybridize with α-synuclein gene. The "hybridizing/binding with/to α-synuclein gene" of the oligonucleotide according to the present invention may be a direct hybridization of the present invention oligonucleotide with α-synuclein gene, a hybridization of the present invention oligonucleotide with mRNA of α-synuclein gene, and a hybridization of the present invention oligonucleotide with mRNA precursor of α-synuclein gene.

The phrase "can hybridize" means that the single strand oligonucleotide can form a structure of double strand nucleic acid due to the nucleobase complementation to a target gene. The index of the thermal stability of binding in the hybridization is not restricted to a melting temperature (Tₘ) of the double strand nucleic acid. For example, a melting temperature (Tₘ) of the double strand nucleic acid can be determined as follows: the oligonucleotide is mixed with equal mole of a target RNA in a buffer solution (8.1 mM Na₂HPO₄, 2.68 mM KCl, 1.47 mM KH₂PO₄, pH 7.2). After the mixture is heated at 95°C for 5 minutes, the mixture is gradually cooled to room temperature for annealing to form a double strand nucleic acid. A temperature of the double strand nucleic acid is increased from 20°C to 95°C at a rate of 0.5°C/min, and a change of an absorbance (A) at 260 nm due to temperature (T) is measured. A graph of dA/dT vs T is drawn on the basis of the measurement result, and Tₘ of the double strand nucleic acid is determined as a temperature at which a value of dA/dT becomes largest, in other words, a temperature at which a change of A due to T becomes largest. For example, the melting temperature (Tₘ) is 40°C or higher and 90°C or lower, and preferably 50°C or higher and 75°C or lower.

The oligonucleotide of the present invention is complementary to α-synuclein gene, but it is not needed that the oligonucleotide is completely complementary to α-synuclein gene and may have a mismatch. For example, it is not needed that base sequences in the region in which the double strand is formed between the present invention oligonucleotide and α-synuclein gene is completely complementary to each other, and there may be 1 or several mismatches as long as the double strand nucleic acid can be formed and a function to suppress the expression is exhibited. The 1 or several mismatches is dependent on the length of the oligonucleotide, and is 1 or more and 4 or less mismatches, preferably 1 or more and 3 or less mismatches, and even more preferably 1 or 2 mismatches. It is preferred that the oligonucleotide of the present invention is completely (100%) complementary to the base sequence in the region at which the double strand is formed.

α-Synuclein (SNCA) gene as a target gene of the present invention oligonucleotide is exemplified by human SNCA ("hSNCA") gene and mouse SNCA ("mSNCA") gene, but is not restricted thereto.

α-Synuclein (SNCA) is a protein composed of 140 amino acid residues and is an amyloid protein which does not have a specific natural structure. α-Synuclein involves an accumulation and a release of synaptic vesicle. The DNA sequence (base sequence) of the coding region of human SNCA (hSNCA) (GenBank accession number: NM_000345) is shown as SEQ ID NO: 1 in Sequence listing. The "SNCA" in the present invention is not restricted to the sequence of SEQ ID NO: 1, and the number and position of mutation of amino acid and DNA are not restricted as long as the function of SNCA protein is retained.

The oligonucleotide of the present invention has an activity to suppress an expression of α-synuclein gene. An activity to suppress an expression (knockdown activity) of SNCA can be measured by a publicly known method. For example, the activity can be measured by transfecting antisense oligonucleotide (ASO) into HEK293A cell as described later or a method of intracerebroventricular administration to an α-synuclein transgenic mouse (SNCA Tg mouse).

An example of the present invention oligonucleotide includes an oligonucleotide having a length of 13 or more and 16 or less nucleotides, preferably an oligonucleotide having a length of 13 or more and 15 or less nucleotides, more preferably an oligonucleotide having a length of 14 or 15 nucleotides. When the oligonucleotide has the above-described length, hybridization with a target SNCA gene, hybridization with mRNA or mRNA precursor of a target SNCA gene, and a suppression of an expression (knockdown) of SNCA may become more effective.

As one embodiment, the oligonucleotide of the present invention has the base sequence described in the following Tables 1-1 and 1-2, and can hybridize with the target region of α-synuclein gene described in Tables 1-1 and 1-2, provided that at least one nucleoside in the sequence is 2'-O,4'-C-ethylene nucleoside. In Tables 1-1 and 1-2, the position of 2'-O,4'-C-ethylene nucleoside is not described and only the base sequence is described. The above-described target region is a region particularly involved in an activity to suppress an expression of α-synuclein gene or a knockdown activity.

**Table 1-1**

| Sequence name | Sequence | Target region in hSNCA gene | | Sequence number |
|---|---|---|---|---|
| | (5'-3') | 5' end position | 3' end position | |
| 40-13 | CCTCCTTGGCCTT | 28 | 40 | 2 |
| 215-13 | GTCACCACTGCTC | 203 | 215 | 3 |
| 227-13 | GCTGTCACACCCG | 215 | 227 | 4 |
| 229-13 | CTGCTGTCACACC | 217 | 229 | 5 |
| 234-13 | GGCTACTGCTGTC | 222 | 234 | 6 |
| 266-13 | GCAATGCTCCCTG | 254 | 266 | 7 |
| 267-13 | TGCAATGCTCCCT | 255 | 267 | 8 |
| 273-13 | GGCTGCTGCAATG | 261 | 273 | 9 |
| 274-13 | TGGCTGCTGCAAT | 262 | 274 | 10 |
| 275-13 | GTGGCTGCTGCAA | 263 | 275 | 11 |
| 277-13 | CAGTGGCTGCTGC | 265 | 277 | 12 |
| 412-13 | GTTCGTAGTCTTG | 400 | 412 | 13 |
| 41-14 | CCCTCCTTGGCCTT | 28 | 41 | 14 |
| 42-14 | TCCCTCCTTGGCCT | 29 | 42 | 15 |
| 74-14 | CCCTGTTTGGTTTT | 61 | 74 | 16 |
| 75-14 | ACCCTGTTTGGTTT | 62 | 75 | 17 |
| 228-14 | TGCTGTCACACCCG | 215 | 228 | 18 |
| 229-14 | CTGCTGTCACACCC | 216 | 229 | 19 |
| 266-14 | GCAATGCTCCCTGC | 253 | 266 | 20 |
| 267-14 | TGCAATGCTCCCTG | 254 | 267 | 21 |
| 268-14 | CTGCAATGCTCCCT | 255 | 268 | 22 |
| 269-14 | GCTGCAATGCTCCC | 256 | 269 | 23 |
| 274-14 | TGGCTGCTGCAATG | 261 | 274 | 24 |
| 275-14 | GTGGCTGCTGCAAT | 262 | 275 | 25 |
| 284-14 | ACAAAGCCAGTGGC | 271 | 284 | 26 |
| 285-14 | GACAAAGCCAGTGG | 272 | 285 | 27 |
| 286-14 | TGACAAAGCCAGTG | 273 | 286 | 28 |
| 366-14 | ATTGTCAGGATCCA | 353 | 366 | 29 |
| 367-14 | CATTGTCAGGATCC | 354 | 367 | 30 |
| 412-14 | GTTCGTAGTCTTGA | 399 | 412 | 31 |
| 413-14 | GGTTCGTAGTCTTG | 400 | 413 | 32 |
| 414-14 | AGGTTCGTAGTCTT | 401 | 414 | 33 |

**Table 1-2**

| Sequence name | Sequence | Target region in hSNCA gene | | Sequence number |
|---|---|---|---|---|
| | (5'-3') | 5' end position | 3' end position | |
| 42-15 | TCCCTCCTTGGCCTT | 28 | 42 | 34 |
| 75-15 | ACCCTGTTTGGTTTT | 61 | 75 | 35 |
| 229-15 | CTGCTGTCACACCCG | 215 | 229 | 36 |
| 254-15 | GCTCCCTCCACTGTC | 240 | 254 | 37 |
| 255-15 | TGCTCCCTCCACTGT | 241 | 255 | 38 |
| 263-15 | ATGCTCCCTGCTCCC | 249 | 263 | 39 |
| 269-15 | GCTGCAATGCTCCCT | 255 | 269 | 40 |
| 274-15 | TGGCTGCTGCAATGC | 260 | 274 | 41 |
| 278-15 | CCAGTGGCTGCTGCA | 264 | 278 | 42 |
| 285-15 | GACAAAGCCAGTGGC | 271 | 285 | 43 |
| 286-15 | TGACAAAGCCAGTGG | 272 | 286 | 44 |
| 288-15 | TTTGACAAAGCCAGT | 274 | 288 | 45 |
| 289-15 | TTTTGACAAAGCCAG | 275 | 289 | 46 |
| 367-15 | CATTGTCAGGATCCA | 353 | 367 | 47 |
| 413-15 | GGTTCGTAGTCTTGA | 399 | 413 | 48 |
| 414-15 | AGGTTCGTAGTCTTG | 400 | 414 | 49 |
| 415-15 | CAGGTTCGTAGTCTT | 401 | 415 | 50 |
| 43-16 | CTCCCTCCTTGGCCTT | 28 | 43 | 51 |
| 76-16 | CACCCTGTTTGGTTTT | 61 | 76 | 52 |
| 230-16 | ACTGCTGTCACACCCG | 215 | 230 | 53 |
| 255-16 | TGCTCCCTCCACTGTC | 240 | 255 | 54 |
| 269-16 | GCTGCAATGCTCCCTG | 254 | 269 | 55 |
| 278-16 | CCAGTGGCTGCTGCAA | 263 | 278 | 56 |
| 286-16 | TGACAAAGCCAGTGGC | 271 | 286 | 57 |
| 368-16 | TCATTGTCAGGATCCA | 353 | 368 | 58 |
| 414-16 | AGGTTCGTAGTCTTGA | 399 | 414 | 59 |

The "target region" in the present invention may be a region on the target SNCA gene, such as the target region of the specified base sequence, for example, the base sequence from the 28^{th} position through the 40^{th} position of SEQ ID NO: 1, and a region on mRNA or mRNA precursor of SNCA gene which mRNA and mRNA precursor correspond to the region on the gene. The phrase "hybridize/bind with/to a target region" means that it is not necessarily needed to form double or more strand (preferably double strand) with the target region as a whole, and the oligonucleotide may form double or more strand (preferably double strand) with the target region partially. The oligonucleotide of the present invention is complementary to, for example, at least a part of a target region and preferably completely complementary to a target region. The term "part" means a region having a length of 13 or more and 15 or less nucleotides in a target region. It is preferred to select a "part" of a target region of which 3' end corresponds to the 40^{th} position of the base sequence of SEQ ID NO: 1 as a target region. The phrase "complementary to at least a part of a target region" may mean that the oligonucleotide is complementary to a base of at least a part of the target region on SNCA gene, such as a region consisting of the base sequence of from the 28^{th} position through the 40^{th} position of SEQ ID NO: 1, or the oligonucleotide is complementary to a base of a region on mRNA or mRNA precursor corresponding to at least the part of the target region.

An example of the preferable base sequence of the present invention oligonucleotide includes a base sequence consisting of a part of a base sequence of the antisense oligonucleotide to a region on mRNA corresponding to the target region described in Table 1 and Table 2. The sequence of the antisense oligonucleotide can be designed by arranging bases complementary to bases of a target region in SEQ ID NO: 1 in a direction from 3' to 5' (3' → 5') by an increment of the number of the base constituting the antisense oligonucleotide (which increment corresponds to the nucleotide length of the oligonucleotide). When a base sequence of the antisense oligonucleotide is described in a direction from 5' to 3', i.e. 5' → 3', the sequence may become reverse complementary sequence to a base sequence of a target region in SEQ ID NO: 1. The oligonucleotide of the present invention may have deletion, substitution, addition or insertion of one or several bases in the above-described sequences as long as the oligonucleotide exhibits an activity to suppress an expression of SNCA. The oligonucleotide may preferably have deletion, substitution, addition or insertion of 1 or more and 3 or less bases, more preferably 1 or 2 bases, and even more preferably 1 base.

Any modifications for a nucleotide publicly known in the technical field except for the above-described sugar modification can be applied to the oligonucleotide of the present invention. A modification of a phosphate and a nucleobase is known as a modification of a nucleotide. Such a nucleic acid modification can be conducted according to a method publicly known in the technical field.

An example of a modification of a phosphate includes S-oligo(phosphorothioate), D-oligo(phosphodiester), M-oligo(methylphosphonate) and boranophosphate at a phosphodiester bond having a natural nucleic acid. The modifications are added into the oligonucleotide according to a publicly known method. S-Oligo(phosphorothioate) has a structure in which a non-bridging oxygen atom in a phosphate group of a phosphodiester bond between nucleosides is substituted by a sulfur atom. One or more, or all of phosphodiester bonds may be changed to a modification of a phosphorothioate.

An example of a nucleobase modification includes 5-methylcytosine, 5-hydroxymethylcytosine and 5-propynylcytosine.

The oligonucleotide of the present invention is preferably a gapmer. A gapmer means an oligonucleotide having a "gap" as a central region and two wings as regions on both side of the gap. Two wings are "5' wing" on the 5' side and "3' wing" on the 3' side.

The gap region of the gapmer of the present invention may have a length of 5 or more and 7 or less nucleotides, preferably a length of 6 or 7 nucleotides, and more preferably a length of 7 nucleotides. The gap is composed of DNA consisting of a natural nucleoside.

The wing region of the gapmer according to the present invention may have a length of 3 or more and 5 or less nucleotides, preferably a length of 3 or 4 nucleotides, and more preferably a length of 3 nucleotides. The oligonucleotide of the present invention contains at least one 2'-O,4'-C-ethylene nucleoside in the "5' wing" and/or "3' wing". The oligonucleotide preferably contains at least one 2'-O,4'-C-ethylene nucleoside in the "5' wing", preferably 1 or more and 4 or less of 2'-O,4'-C-ethylene nucleosides, more preferably 2 or more and 4 or less 2'-O,4'-C-ethylene nucleosides, even more preferably two or three 2'-O,4'-C-ethylene nucleosides, and particularly preferably two 2'-O,4'-C-ethylene nucleosides, and the oligonucleotide preferably contains at least one 2'-O,4'-C-ethylene nucleoside in the "3' wing", preferably 1 or more and 4 or less 2'-O,4'-C-ethylene nucleosides, more preferably 2 or more and 4 or less 2'-O,4'-C-ethylene nucleosides, even more preferably two or three 2'-O,4'-C-ethylene nucleosides, and particularly preferably two 2'-O,4'-C-ethylene nucleosides.

As one embodiment, the oligonucleotide is composed of a gap region of 5 or more and 7 or less nucleotides, 5' wing of 3 or more and 5 or less nucleotides, and 3' wing of 3 or more and 5 or less nucleotides, wherein the gap region is placed between the 5' wing and 3' wing, and the 5' wing and 3' wing may contain at least one 2'-O,4'-C-ethylene nucleoside. In addition, the oligonucleotide may contain a modification of a phosphate and a base. A kind, number and position of a modification in one of the wings may be the same as or different from a kind, number and position of a modification in the other wing.

As one embodiment, the oligonucleotide is composed of a gap region of 5 or more and 7 or less of nucleotides, 5' wing of 3 nucleotides, and 3' wing of 3 nucleotides, wherein the 5' wing and 3' wing may respectively contain at least one 2'-O,4'-C-ethylene nucleoside.

As one embodiment, the oligonucleotide is composed of a gap region of 6 or 7 nucleotides, 5' wing of 3 nucleotides, and 3' wing of 3 nucleotides, wherein 2 of 3 in the 5' wing are 2'-O,4'-C-ethylene nucleosides, and 2 of 3 in the 3' wing may contain 2'-O,4'-C-ethylene nucleoside.

An example of such a gapmer includes 3-7-3, 4-6-3, 3-6-4, 4-5-4, 4-7-3, 3-7-4, 4-6-4, 5-6-3, 3-6-5, 3-7-5, 5-7-3, 4-7-4, 4-6-5, 5-6-4, 5-5-5 and 5-6-5. In the description of "A-B-C" or "A-B-C-D", "A" represents the number of base in the 5' wing, "B" represents the number of base in the gap, "C" represents the number of sugar-modified nucleoside in a base which forms the 3' wing, and "D" represents the number of natural nucleoside in a base which forms the 3' wing. For example, in the case of the description of 3-7-3, 3-7-3 is composed of the gap consisting of 7 natural nucleotides (DNA), 5' wing consisting of 3 nucleotides from the 5' end, and 3' wing consisting of 3 nucleotides from the 3' end, wherein the 5' wing and 3' wing may respectively contain at least one 2'-O,4'-C-ethylene nucleoside.

The 5' wing and 3' wing may contain a nucleoside modified by 2'-O-alkylated nucleoside, AmNA described in a document (Yahara, A. et al., ChemBioChem, (2012), 13, 2513-2516) or WO 2014/109384, or S-cEt (2',4'-constrained ethyl). The number of a modification is not particularly restricted and may be appropriately adjusted for any purpose. Two or more 2'-O-alkylated nucleosides, AmNA or S-cEt may be the same as or different from each other.

As 2'-O-alkylated nucleoside, 2'-O-alkylated D-ribofuranose such as 2'-O-methylated, 2'-O-aminoethylated, 2'-O-propylated, 2'-O-allylated, 2'-O-methoxyethylated, 2'-O-butylated, 2'-O-pentylated and 2'-O-propargylated D-ribofuranose may be used.

An example of the oligonucleotide according to the present invention includes oligonucleotides of Examples 23, 28, 29, 32 and 170. The sequences may have one or several deletions, substitutions, additions or insertions of bases as long as the oligonucleotide exhibits an activity to suppress an expression of SNCA. The oligonucleotide may be an oligonucleotide preferably having 1 or more and 3 or less, more preferably 1 or 2, and even more preferably 1 deletion, substitution, addition or insertion of base.

The oligonucleotide of the present invention can be synthesized by an ordinary method from the above-described sugar-modified nucleoside and natural nucleoside. For example, the oligonucleotide can be easily synthesized by using a commercially available automated nucleic acid synthesizer manufactured by, for example, BioAutomation, Applied Biosystems or GeneDesign. A synthesis method is exemplified by solid-phase synthesis method using phosphoramidite and solid-phase synthesis method using hydrogen phosphonate. For example, the synthesis method is disclosed in Tetrahedron Letters, 1981, vol. 22, pp. 1859-1862, WO 2011/052436 or the like.

The present invention also relates to an α-synuclein expression inhibitor comprising the oligonucleotide of the present invention. In this disclosure, the "a-synuclein expression inhibitor" suppresses a biosynthesis of α-synuclein by hybridizing with α-synuclein gene to suppress an expression of α-synuclein gene. The present invention further relates to a pharmaceutical composition comprising the oligonucleotide of the present invention. An administration method and a formulation of the α-synuclein expression inhibitor and pharmaceutical composition of the present invention may be an administration method and a formulation publicly known in the technical field.

The pharmaceutical composition of the present invention can be administered by various methods depending on whether topical administration or systemic administration, or a lesion to be treated. The pharmaceutical composition may be administered topically (including instillationally, intravaginally, intrarectally, intranasally and dermally), orally or parenterally. An example of parenteral administration includes intravenous infusion or drip infusion; subcutaneous, intraperitoneal or intramuscular injection; lung administration by aspiration or inhalation; intrathecal administration; and intracerebroventricular administration.

When the pharmaceutical composition of the present invention is topically administered, a formulation such as transdermal patch, ointment, lotion, cream, gel, aqueous drip formulation, suppository, aerosolized formulation, liquid formulation and powder formulation can be used.

An example of the composition for oral administration includes powder formulation, granule formulation, dispersion or solution of water or non-aqueous medium, capsule, powder formulation and tablet.

An example of the composition for parenteral administration, intrathecal administration or intracerebroventricular administration includes an abacterial aqueous solution including buffer, diluent and other appropriate additive.

The pharmaceutical composition of the present invention can be obtained by mixing an effective amount of the oligonucleotide according to the present invention with various pharmaceutical additives suitable for the dosage form as appropriate. An example of the pharmaceutical additive includes excipient, binder, moisturizer, disintegrant, lubricant and diluent. In the case of an injectable formulation, the oligonucleotide with an appropriate carrier is sterilized to be a formulation.

An example of an excipient includes lactose, sucrose, glucose, starch, calcium carbonate and crystalline cellulose. An example of a binder includes methylcellulose, carboxymethylcellulose, hydroxypropylcellulose, gelatin and polyvinylpyrrolidone. An example of a disintegrant includes carboxymethylcellulose, carboxymethylcellulose sodium, starch, sodium alginate, agar powder and sodium lauryl sulfate. An example of a lubricant includes talc, magnesium stearate and macrogol. An example of a base material of a suppository includes cacao butter, macrogol and methylcellulose. When the composition is produced as a liquid formulation or an emulsion or suspension injectable formulation, usually used solubilizing agent, suspending agent, emulsifier, stabilizing agent, preserving agent, isotonic agent or the like may be appropriately added. A flavoring agent, fragrance or the like may be added in the case of oral administration.

The pharmaceutical composition of the present invention can be used for treating or preventing a disease involving α-synuclein (SNCA) gene. For example, the pharmaceutical composition of the present invention can be used for medical treatment or prevention on the basis of an activity to suppress an expression of SNCA (knockdown activity). A disease for which the pharmaceutical composition of the present invention is used is exemplified by α-synuclein excess symptom. It can be expected to prevent a progression of nerve degeneration and an onset of dementia, particularly DLB, by an activity to suppress an expression of SNCA (knockdown activity) of the pharmaceutical composition according to the present invention. For example, the pharmaceutical composition of the present invention can be used for treating or preventing Parkinson's disease or Lewy body dementia.

The present invention provides a method for suppressing an expression of α-synuclein. Also, the present invention provides a method for treating and preventing α-synuclein excess symptom, and a method for treating and preventing Parkinson's disease or Lewy body dementia. The methods comprise the step of administering the oligonucleotide of the present invention to a subject. The term "subject" is preferably a mammal, more preferably human, monkey, dog, cat, rat and mouse, and even more preferably human. An administration method and a dosage form are not restricted in the above-described methods as long as an effective amount of the oligonucleotide according to the present invention is administered. An effective administration amount is dependent on a subject to whom the oligonucleotide is administered and can be arbitrarily adjusted depending on sex, age, body weight, symptom or the like of the subject, and method, route, frequency or the like of administration. For example, a dose amount can be adjusted to 0.1 mg/kg or more and 10 mg/kg or less. An administration method or the like is described above.

The present application claims the benefit of the priority date of Japanese patent application No. 2017-132290 filed on July 5, 2017. All of the contents of the Japanese patent application No. 2017-132290 filed on July 5, 2017, are incorporated by reference herein.

### EXAMPLES

Hereinafter, the present invention is specifically described with Examples. The Examples are intended for use in the explanation of the resent invention and do not restrict the scope of the present invention.

### Example 1: Synthesis of HO-C^{e2s}-C^{m1s}-T^{e2s}-5meC^{s}-5meC^{s}-T^{s}-T^{s}-G^{s}-G^{s}-5meC^{s}-C^{e2s}-T^{m1s}-T^{e2t}-H (40-13A) (SEQ ID NO: 2)

The title oligonucleotide was synthesized by phosphoramidite method (Nucleic Acids Research, 12, 4539 (1984)) using an automated nucleic acid synthesizer ("MerMade 192X" manufactured by BioAutomation). A solution prepared by adding 0.4% of 1-methylimidazole (manufactured by Wako Pure Chemical Industries, Ltd., product No. 134-12801) to an activator solution-3 (0.25 mol/L 5-benzylthio-1H-tetrazole acetonitrile solution, manufactured by Wako Pure Chemical Industries, Ltd., product No. 013-20011) was used for the condensation, and the reaction time was set to about 10 minutes. Phenylacetyl Disulfide (manufactured by CARBOSYNTH, product No. FP07495) was dissolved in a mixed solvent of dehydrated acetonitrile (manufactured by KANTO CHEMICAL CO., INC., product No. 01837-05) : dehydrated pyridine (manufactured by KANTO CHEMICAL CO., INC., product No. 11339-05) = 1 : 1 (v/v) in a concentration of 0.2 M, and the solution was used as a thioation reagent to form a phosphorothioate bond. Other used reagents were CAP A for AKTA (1-methylimidazole·acetonitrile solution, manufactured by Sigma-Aldrich, product No. L040050), Cap B1 for AKTA (acetic anhydride·acetonitrile solution, manufactured by Sigma-Aldrich, product No. L050050), Cap B2 for AKTA (pyridine·acetonitrile solution, manufactured by Sigma-Aldrich, product No. L050150), DCA Deblock (dichloroacetic acid·toluene solution, manufactured by Sigma-Aldrich, product No. L023050). Phosphoramidites of 2'-O-Me nucleoside (adenosine product No. ANP-5751, cytidine product No. ANP-5752, guanosine product No. ANP-5753, uridine product No. ANP-5754) manufactured by ChemGenes were used as amidite reagents. The compounds of Example 14 (5'-O-dimethoxytrityl-2'-O,4'-C-ethylene-6-N-benzoyladenosine-3'-O-(2-cyanoethyl N,N-diisopropyl)phosphoramidite), Example 27 (5'-O-dimethoxytrityl-2'-O,4'-C-ethylene-2-N-isobutyrylguanosine-3'-O-(2-cyanoethyl N,N-diisopropyl)phosphoramidite), Example 22 (5'-O-dimethoxytrityl-2'-O,4'-C-ethylene-4-N-benzoyl-5-methylcytidine-3'-O-(2-cyanoethyl N,N-diisopropyl)phosphoramidite), Example 9 (5'-O-dimethoxytrityl-2'-O,4'-C-ethylene-5-methyluridine-3'-O-(2-cyanoethyl N,N-diisopropyl)phosphoramidite) described in JP 2000-297097 A were used as unnatural phosphoramidites. The title compound was synthesized by using Glen Unysupport FC 96 well format 0.2 µmol (manufactured by GlenResearch) as a solid phase support. The cyanoethyl group as the protective group on the phosphorus atom and the protective group on the nucleobase were removed while an oligomer was cut out from the support by treating the protected oligonucleotide analogue having the objective sequence with 600 µL of concentrated ammonia water. The solution containing the oligomer was mixed with 300 µL of Clarity QSP DNA Loading Buffer (manufactured by Phenomenex), and the mixture was charged on Clarity SPE 96 well plate (manufactured by Phenomenex). After 1 mL of a solution of Clarity QSP DNA Loading Buffer : water = 1 : 1, 2 mL of a solution of 0.1 M tetrabutylammonium bromide aqueous solution : acetonitrile = 8 : 2 (v/v), 3 mL of 3% dichloroacetic acid (DCA) aqueous solution, 4 mL of water, and 2 mL of 20 mM Tris aqueous solution were added in this order, and components extracted by a solution of 20 mM Tris aqueous solution : acetonitrile = 9 : 1 were collected. The solvent was distilled away to obtain the target compound. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 2.849 minutes. The compound was identified by negative ion ESI mass spectrometric analysis (calculated value: 4326.51, measured value: 4326.51).

The base sequence of the compound is complementary to nucleotide number 28 to 40 in the coding region of Homo sapiens synuclein, alpha (SNCA), transcript variant 1, mRNA (NCBI-GenBank accession No.NM_000345).

The base sequences are described in Sequence listing without distinguishing natural nucleoside and 2'-O,4'-C-ethylene nucleoside.

### Examples 2 to 12

The compounds of Examples 2 to 12 described in Table 2 were synthesized similarly to Example 1.

**Table 2**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 1 | 40-13A | CcTCCTTGGCCuT | 28 | 40 | 4326.51 | 2 |
| 2 | 215-13A | GuCACCACTGCuC | 203 | 215 | 4344.53 | 3 |
| 3 | 227-13A | GcTGTCACACCcG | 215 | 227 | 4369.54 | 4 |
| 4 | 229-13A | CuGCTGTCACAcC | 217 | 229 | 4344.53 | 5 |
| 5 | 234-13A | GgCTACTGCTGuC | 222 | 234 | 4401.51 | 6 |
| 6 | 266-13A | GcAATGCTCCCuG | 254 | 266 | 4370.52 | 7 |
| 7 | 267-13A | TgCAATGCTCCcT | 255 | 267 | 4359.52 | 8 |
| 8 | 273-13A | GgCTGCTGCAAuG | 261 | 273 | 4436.51 | 9 |
| 9 | 274-13A | TgGTGCTGCAaT | 262 | 274 | 4425.52 | 10 |
| 10 | 275-13A | GuGGCTGCTGCaA | 263 | 275 | 4436.52 | 11 |
| 11 | 277-13A | CaGTGGCTGCTgC | 265 | 277 | 4440.53 | 12 |
| 12 | 412-13A | GuTCGTAGTCTuG | 400 | 412 | 4389.46 | 13 |

In the sequences in the table, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Each nucleoside was bound through phosphorothioate. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 13: Synthesis of HO-G^{e2s}-C^{m1s}-T^{e2s}-G^{m1s}-T^{s}-5meC^{s}-A^{s}-5meC^{s}-A^{s}-5meC^{s}-C^{e2s}-C^{m1s}-G^{e2t}-H (227-13B) (SEQ ID NO: 4)

The target compound was obtained by synthesis and purification in a similar condition to Example 1. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 2.326 minutes. The compound was identified by negative ion ESI mass spectrometric analysis (calculated value: 4399.55, measured value: 4399.54).

The base sequence of the compound is complementary to nucleotide number 215 to 227 in the coding region of Homo sapiens synuclein, alpha (SNCA), transcript variant 1, mRNA (NCBI-GenBank accession No.NM_000345).

### Examples 14 to 20

The compounds of Examples 14 to 20 described in Table 3 were synthesized similarly to Example 13.

**Table 3**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 13 | 227-13B | GcTgTCACACCcG | 215 | 227 | 4399.54 | 4 |
| 14 | 227-13D | GcTgTCACAcCcG | 215 | 227 | 4415.52 | 4 |
| 15 | 234-13C | GgCTACTGCuGuC | 222 | 234 | 4417.50 | 6 |
| 16 | 266-13D | GcAaTGCTCcCuG | 254 | 266 | 4416.51 | 7 |
| 17 | 273-13C | GgCTGCTGCaAuG | 261 | 273 | 4466.50 | 9 |
| 18 | 275-13B | GuGgCTGCTGCaA | 263 | 275 | 4466.49 | 11 |
| 19 | 275-13D | GuGgCTGCTgCaA | 263 | 275 | 4496.50 | 11 |
| 20 | 277-13B | CaGuGGCTGCTgC | 265 | 277 | 4456.46 | 12 |

In the sequences in Table 3, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Each nucleoside was bound through phosphorothioate. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 21: Synthesis of HO-T^{e2s}-C^{m1s}-C^{e2s}-C^{m1s}-T^{s}-5meC^{s}-5meC^{s}-T^{s}-T^{s}-G^{s}-G^{s}-C^{m1s}-C^{e2s}-U^{m1s}-T^{e2t}-H (42-15A) (SEQ ID NO: 34)

The target compound was obtained by synthesis and purification in a similar condition to Example 1. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 3.01 minutes. The compound was identified by negative ion ESI mass spectrometric analysis (calculated value: 4997.56, measured value: 4997.55).

The base sequence of the compound is complementary to nucleotide number 28 to 42 in the coding region of Homo sapiens synuclein, alpha (SNCA), transcript variant 1, mRNA (NCBI-GenBank accession No.NM_000345).

### Examples 22 to 34

The compounds of Examples 22 to 34 described in Table 4 were synthesized similarly to Example 21.

**Table 4**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 21 | 42-15A | TcCcTCCTTGGcCuT | 28 | 42 | 4997.55 | 34 |
| 22 | 75-15A | AcCcTGTTTGGuTuT | 61 | 75 | 5035.51 | 35 |
| 23 | 229-15A | CuGcTGTCACAcCcG | 215 | 229 | 5040.58 | 36 |
| 24 | 254-15A | GcTcCCTCCACuGuC | 240 | 254 | 5005.57 | 37 |
| 25 | 255-15A | TgCuCCCTCCAcTgT | 241 | 255 | 5034.59 | 38 |
| 26 | 269-15A | GcTgCAATGCTcCcT | 255 | 269 | 5055.57 | 40 |
| 27 | 274-15A | TgGcTGCTGCAaTgC | 260 | 274 | 5135.59 | 41 |
| 28 | 278-15A | CcAgTGGCTGCuGcA | 264 | 278 | 5106.57 | 42 |
| 29 | 285-15A | GaCaAAGCCAGuGgC | 271 | 285 | 5162.63 | 43 |
| 30 | 286-15A | TgAcAAAGCCAgTgG | 272 | 286 | 5163.62 | 44 |
| 31 | 367-15A | CaTuGTCAGGAuCcA | 353 | 367 | 5075.57 | 47 |
| 32 | 413-15A | GgTuCGTAGTCuTgA | 399 | 413 | 5123.54 | 48 |
| 33 | 414-15A | AgGuTCGTAGTcTuG | 400 | 414 | 5109.52 | 49 |
| 34 | 415-15A | CaGgTTCGTAGuCuT | 401 | 415 | 5097.55 | 50 |

In the sequences in Table 4, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Each nucleoside was bound through phosphorothioate. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 35: Synthesis of HO-A^{e2s}-U^{m1s}-G^{e2s}-C^{m1s}-T^{s}-5meC^{s}-5meC^{s}-5meC^{s}-T^{s}-G^{s}-5meC^{s}-U^{m1s}-C^{e2s}-C^{m1s}-C^{e2t}-H (263-15A) (SEQ ID NO: 39)

The target compound shown in Table 5 was obtained by synthesis and purification in a similar condition to Example 1. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 2.969 minutes. The compound was identified by negative ion ESI mass spectrometric analysis (calculated value: 5005.59, measured value: 5005.58).

The base sequence of the compound is complementary to nucleotide number 249 to 263 in the coding region of Homo sapiens synuclein, alpha (SNCA), transcript variant 1, mRNA (NCBI-GenBank accession No.NM_000345).

### Examples 36 and 37

The compounds of Examples 36 and 37 shown in Table 5 were synthesized similarly to Example 35.

**Table 5**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 35 | 263-15A | AuGcTCCCTGCuCcC | 249 | 263 | 5005.58 | 39 |
| 36 | 288-15A | TuTgACAAAGCcAgT | 274 | 288 | 5099.60 | 45 |
| 37 | 289-15A | TuTuGACAAAGcCaG | 275 | 289 | 5085.57 | 46 |

In the sequences in Table 5, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Each nucleoside was bound through phosphorothioate. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 38: Synthesis of HO-T^{e2s}-C^{m1s}-C^{e2s}-C^{m1s}-T^{e2s}-5meC^{s}-5meC^{s}-T^{s}-T^{s}-G^{s}-G^{s}-C^{m1s}-C^{e2s}-U^{m1s}-T^{e2t}-H (42-15B) (SEQ ID NO: 34)

The target compound shown in Table 6 was obtained by synthesis and purification in a similar condition to Example 1. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 2.750 minutes. The compound was identified by negative ion ESI mass spectrometric analysis (calculated value: 5039.57, measured value: 5039.59).

The base sequence of the compound is complementary to nucleotide number 28 to 42 in the coding region of Homo sapiens synuclein, alpha (SNCA), transcript variant 1, mRNA (NCBI-GenBank accession No.NM_000345).

### Examples 39 to 88

The compounds of Examples 39 to 88 shown in Table 6 were synthesized similarly to Example 38.

**Table 6**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 38 | 42-15B | TcCcTCCTTGGcCuT | 28 | 42 | 5039.59 | 34 |
| 39 | 42-15C | TcCcTCCTTGGcCuT | 28 | 42 | 5039.58 | 34 |
| 40 | 42-15D | TcCcTCCTTGGcCuT | 28 | 42 | 5081.59 | 34 |
| 41 | 75-15B | AcCcTGTTTGGuTuT | 61 | 75 | 5077.54 | 35 |
| 42 | 75-15C | AcCcTGTTTGGuTuT | 61 | 75 | 5077.52 | 35 |
| 43 | 75-15D | AcCcTGTTTGGuTuT | 61 | 75 | 5119.57 | 35 |
| 44 | 229-15B | CuGcTGTCACAcCcG | 215 | 229 | 5082.60 | 36 |
| 45 | 229-15C | CuGcTGTCACAcCcG | 215 | 229 | 5082.60 | 36 |
| 46 | 229-15D | CuGcTGTCACAcCcG | 215 | 229 | 5124.60 | 36 |
| 47 | 254-15B | GcTcCCTCCACuGuC | 240 | 254 | 5047.61 | 37 |
| 48 | 254-15C | GcTcCCTCCACuGuC | 240 | 254 | 5047.61 | 37 |
| 49 | 254-15D | GcTcCCTCCACuGuC | 240 | 254 | 5089.66 | 37 |
| 50 | 255-15B | TgCuCCCTCCAcTgT | 241 | 255 | 5076.60 | 38 |
| 51 | 255-15C | TgCuCCCTCCAcTgT | 241 | 255 | 5076.61 | 38 |
| 52 | 255-15D | TgCuCCCTCCAcTgT | 241 | 255 | 5118.62 | 38 |
| 53 | 263-15B | AuGcTCCCTGCuCcC | 249 | 263 | 5047.59 | 39 |
| 54 | 263-15C | AuGcTCCCTGCuCcC | 249 | 263 | 5047.57 | 39 |
| 55 | 263-15D | AuGcTCCCTGCuCcC | 249 | 263 | 5089.60 | 39 |
| 56 | 269-15B | GcTgCAATGCTcCcT | 255 | 269 | 5097.60 | 40 |
| 57 | 269-15C | GcTgCAATGCTcCcT | 255 | 269 | 5097.60 | 40 |
| 58 | 269-15D | GcTgCAATGCTcCcT | 255 | 269 | 5139.62 | 40 |
| 59 | 274-15B | TgGcTGCTGCAaTgC | 260 | 274 | 5177.60 | 41 |
| 60 | 274-15C | TgGcTGCTGCAaTgC | 260 | 274 | 5177.62 | 41 |
| 61 | 274-15D | TgGcTGCTGCAaTgC | 260 | 274 | 5219.63 | 41 |
| 62 | 278-15B | CcAgTGGCTGCuGcA | 264 | 278 | 5148.91 | 42 |
| 63 | 278-15C | CcAgTGGCTGCuGcA | 264 | 278 | 5148.96 | 42 |
| 64 | 278-15D | CcAgTGGCTGCuGcA | 264 | 278 | 5190.60 | 42 |
| 65 | 285-15B | GaCaAAGCCAGuGgC | 271 | 285 | 5204.66 | 43 |
| 66 | 285-15C | GaCaAAGCCAGuGgC | 271 | 285 | 5204.65 | 43 |
| 67 | 285-15D | GaCaAAGCCAGuGgC | 271 | 285 | 5246.66 | 43 |
| 68 | 286-15B | TgAcAAAGCCAgTgG | 272 | 286 | 5204.64 | 44 |
| 69 | 286-15C | TgAcAAAGCCAgTgG | 272 | 286 | 5205.64 | 44 |
| 70 | 286-15D | TgAcAAAGCCAgTgG | 272 | 286 | 5247.64 | 44 |
| 71 | 288-15B | TuTgACAAAGCcAgT | 274 | 288 | 5141.61 | 45 |
| 72 | 288-15C | TuTgACAAAGCcAgT | 274 | 288 | 5141.60 | 45 |
| 73 | 288-15D | TuTgACAAAGCcAgT | 274 | 288 | 5183.61 | 45 |
| 74 | 289-15B | TuTuGACAAAGcCaG | 275 | 289 | 5127.58 | 46 |
| 75 | 289-15C | TuTuGACAAAGcCaG | 275 | 289 | 5127.58 | 46 |
| 76 | 289-15D | TuTuGACAAAGcCaG | 275 | 289 | 5169.79 | 46 |
| 77 | 367-15B | CaTuGTCAGGAuCcA | 353 | 367 | 5117.59 | 47 |
| 78 | 367-15C | CaTuGTCAGGAuCcA | 353 | 367 | 5117.59 | 47 |
| 79 | 367-15D | CaTuGTCAGGAuCcA | 353 | 367 | 5159.60 | 47 |
| 80 | 413-15B | GgTuCGTAGTCuTgA | 399 | 413 | 5165.61 | 48 |
| 81 | 413-15C | GgTuCGTAGTCuTgA | 399 | 413 | 5165.58 | 48 |
| 82 | 413-15D | GgTuCGTAGTCuTgA | 399 | 413 | 5207.96 | 48 |
| 83 | 414-15B | AgGuTCGTAGTcTuG | 400 | 414 | 5151.54 | 49 |
| 84 | 414-15C | AgGuTCGTAGTcTuG | 400 | 414 | 5151.54 | 49 |
| 85 | 414-15D | AgGuTCGTAGTcTuG | 400 | 414 | 5193.55 | 49 |
| 86 | 415-15B | CaGgTTCGTAGuCuT | 401 | 415 | 5139.59 | 50 |
| 87 | 415-15C | CaGgTTCGTAGuCuT | 401 | 415 | 5139.57 | 50 |
| 88 | 415-15D | CaGgTTCGTAGuCuT | 401 | 415 | 5181.64 | 50 |

In the sequences in Table 6, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Each nucleoside was bound through phosphorothioate. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 89: Synthesis of HO-C^{e2s}-C^{m1s}-C^{e2s}-U^{m1s}-5meC^{s}-5meC^{s}-T^{s}-T^{s}-G^{s}-G^{s}-C^{m1s}-C^{e2s}-U^{m1s}-T^{e2t}-H (41-14A) (SEQ ID NO: 14)

The target compound shown in Table 7 was obtained by synthesis and purification in a similar condition to Example 1. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 3.101 minutes. The compound was identified by negative ion ESI mass spectrometric analysis (calculated value: 4677.54, measured value: 4677.54).

The base sequence of the compound is complementary to nucleotide number 28 to 41 in the coding region of Homo sapiens synuclein, alpha (SNCA), transcript variant 1, mRNA (NCBI-GenBank accession No.NM_000345).

### Examples 90 to 108

The compounds of Examples 90 to 108 shown in Table 7 were synthesized similarly to Example 89. The data of Examples 89 to 108 are shown in Table 7.

**Table 7**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 89 | 41-14A | CcCuCCTTGGcCuT | 28 | 41 | 4677.54 | 14 |
| 90 | 42-14A | TcCcTCCTTGgCcT | 29 | 42 | 4691.57 | 15 |
| 91 | 74-14A | CcCuGTTTGGuTuT | 61 | 74 | 4706.49 | 16 |
| 92 | 75-14A | AcCcTGTTTGgTuT | 62 | 75 | 4729.52 | 17 |
| 93 | 228-14A | TgCuGTCACAcCcG | 215 | 228 | 4735.56 | 18 |
| 94 | 229-14A | CuGcTGTCACaCcC | 216 | 229 | 4709.57 | 19 |
| 95 | 266-14A | GcAaTGCTCCcTgC | 253 | 266 | 4749.59 | 20 |
| 96 | 267-14A | TgCaATGCTCcCuG | 254 | 267 | 4750.56 | 21 |
| 97 | 268-14A | CuGcAATGCTcCcT | 255 | 268 | 4696.54 | 22 |
| 98 | 269-14A | GcTgCAATGCuCcC | 256 | 269 | 4735.57 | 23 |
| 99 | 274-14A | TgGcTGCTGCaAuG | 261 | 274 | 4802.55 | 24 |
| 100 | 275-14A | GuGgCTGCTGcAaT | 262 | 275 | 4802.54 | 25 |
| 101 | 284-14A | AcAaAGCCAGuGgC | 271 | 284 | 4803.60 | 26 |
| 102 | 285-14A | GaCaAAGCCAgTgG | 272 | 285 | 4857.62 | 27 |
| 103 | 286-14A | TgAcAAAGCCaGuG | 273 | 286 | 4804.58 | 28 |
| 104 | 366-14A | AuTgTCAGGAuCcA | 353 | 366 | 4756.54 | 29 |
| 105 | 367-14A | CaTuGTCAGGaTcC | 354 | 367 | 4760.55 | 30 |
| 106 | 412-14A | GuTcGTAGTCuTgA | 399 | 412 | 4764.50 | 31 |
| 107 | 413-14A | GgTuCGTAGTcTuG | 400 | 413 | 4780.51 | 32 |
| 108 | 414-14A | AgGuTCGTAGuCuT | 401 | 414 | 4764.51 | 33 |

In the sequences in Table 7, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Each nucleoside was bound through phosphorothioate. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 109: Synthesis of HO-C^{e2s}-U^{m1s}-C^{e2s}-C^{m1s}-C^{e2s}-T^{s}-5meC^{s}-5meC^{s}-T^{s}-T^{s}-G^{s}-G^{e2s}-C^{m1s}-C^{e2s}-U^{m1s}-T^{e2t}-H (43-16A) (SEQ ID NO: 51)

The target compound shown in Table 8 was obtained by synthesis and purification in a similar condition to Example 1. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 3.132 minutes. The compound was identified by negative ion ESI mass spectrometric analysis (calculated value: 5400.62, measured value: 5400.61).

The base sequence of the compound is complementary to nucleotide number 28 to 43 in the coding region of Homo sapiens synuclein, alpha (SNCA), transcript variant 1, mRNA (NCBI-GenBank accession No.NM_000345).

### Examples 110 to 117

The compounds of Examples 110 to 117 shown in Table 8 were synthesized similarly to Example 109.

**Table 8**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 109 | 43-16A | CuCcCTCCTTGGcCuT | 28 | 43 | 5400.61 | 51 |
| 110 | 76-16A | CaCcCTGTTTGGuTuT | 61 | 76 | 5452.60 | 52 |
| 111 | 230-16A | AcTgCTGTCACAcCcG | 215 | 230 | 5467.67 | 53 |
| 112 | 255-16A | TgCuCCCTCCACuGuC | 240 | 255 | 5423.65 | 54 |
| 113 | 269-16A | GcTgCAATGCTCcCuG | 254 | 269 | 5484.65 | 55 |
| 114 | 278-16A | CcAgTGGCTGCTgCaA | 263 | 278 | 5547.69 | 56 |
| 115 | 286-16A | TgAcAAAGCCAGuGgC | 271 | 286 | 5552.67 | 57 |
| 116 | 368-16A | TcAuTGTCAGGAuCcA | 353 | 368 | 5465.61 | 58 |
| 117 | 414-16A | AgGuTCGTAGTCuTgA | 399 | 414 | 5536.60 | 59 |

In the sequences in Table 8, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Each nucleoside was bound through phosphorothioate. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 118: Synthesis of HO-C^{m1s}-C^{e2s}-T^{e2s}-5meC^{s}-5meC^{s}-T^{s}-T^{s}-G^{s}-G^{s}-5meC^{s}-C^{e2s}-T^{e2s}-U^{m1t}-H (40-13E) (SEQ ID NO: 2)

The target compound shown in Table 9 was obtained by synthesis and purification in a similar condition to Example 1. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 2.509 minutes. The compound was identified by negative ion ESI mass spectrometric analysis (calculated value: 4326.51, measured value: 4326.51).

The base sequence of the compound is complementary to nucleotide number 28 to 43 in the coding region of Homo sapiens synuclein, alpha (SNCA), transcript variant 1, mRNA (NCBI-GenBank accession No.NM_000345).

### Examples 119 to 129

The compounds of Examples 119 to 129 shown in Table 9 were synthesized similarly to Example 118.

**Table 9**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 118 | 40-13E | cCTCCTTGGCCTu | 28 | 40 | 4326.51 | 2 |
| 119 | 215-13E | gTCACCACTGCTc | 203 | 215 | 4358.55 | 3 |
| 120 | 227-13E | gCTGTCACACCCg | 215 | 227 | 4397.57 | 4 |
| 121 | 229-13E | cTGCTGTCACACc | 217 | 229 | 4344.54 | 5 |
| 122 | 234-13E | gGCTACTGCTGTc | 222 | 234 | 4401.52 | 6 |
| 123 | 266-13E | gCAATGCTCCCTg | 254 | 266 | 4398.56 | 7 |
| 124 | 267-13E | uGCAATGCTCCCu | 255 | 267 | 4345.51 | 8 |
| 125 | 273-13E | gGCTGCTGCAATg | 261 | 273 | 4450.54 | 9 |
| 126 | 274-13E | uGGCTGCTGCAAu | 262 | 274 | 4397.49 | 10 |
| 127 | 275-13E | gTGGCTGCTGCAa | 263 | 275 | 4450.54 | 11 |
| 128 | 277-13E | cAGTGGCTGCTGc | 265 | 277 | 4412.51 | 12 |
| 129 | 412-13E | gTTCGTAGTCTTg | 400 | 412 | 4417.50 | 13 |

In the sequences in Table 9, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Each nucleoside was bound through phosphorothioate. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 130: Synthesis of HO-C^{e3s}-C^{m1s}-T^{e3s}-C^{s}-C^{s}-T^{s}-T^{s}-G^{s}-G^{s}-C^{s}-C^{e3s}-U^{m1s}-T^{e2t}-H (40-13F) (SEQ ID NO: 2)

The synthesis was conducted in accordance with phosphoramidite method (Nucleic Acids Research, 12, 4539 (1984)) using an automated nucleic acid synthesizer. A phosphoramidite of AmNA was synthesized with reference to WO 2011/052436.

The base sequence of the compound is complementary to nucleotide number 28 to 40 in the coding region of Homo sapiens synuclein, alpha (SNCA), transcript variant 1, mRNA (NCBI-GenBank accession No.NM_000345).

### Examples 131 to 164

The compounds of Examples 131 to 164 shown in Table 10 were synthesized similarly to Example 130.

**Table 10**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 130 | 40-13F | cctCCTTGGCcuT | 28 | 40 | 4324.54 | 2 |
| 131 | 215-13F | gucACCACTGcuC | 203 | 215 | 4344.07 | 3 |
| 132 | 227-13F | gctGTCACACccG | 215 | 227 | 4366.20 | 4 |
| 133 | 229-13F | cugCTGTCACacC | 217 | 229 | 4341.62 | 5 |
| 134 | 234-13F | ggcTACTGCTguC | 222 | 234 | 4412.35 | 6 |
| 135 | 266-13F | gcaATGCTCCcuG | 254 | 266 | 4366.02 | 7 |
| 136 | 267-13F | tgcAATGCTCccT | 255 | 267 | 4371.16 | 8 |
| 137 | 273-13F | ggcTGCTGCAauG | 261 | 273 | 4446.33 | 9 |
| 138 | 274-13F | tggCTGCTGCaaT | 262 | 274 | 4422.33 | 10 |
| 139 | 275-13F | gugGCTGCTGcaA | 263 | 275 | 4446.69 | 11 |
| 140 | 277-13F | cagTGGCTGCtgC | 265 | 277 | 4454.49 | 12 |
| 141 | 412-13F | gutCGTAGTCtuG | 400 | 412 | 4400.08 | 13 |
| 142 | 227-13G | gctgTCACACccG | 215 | 227 | 4394.13 | 4 |
| 143 | 227-13H | gctGTCACAcccG | 215 | 227 | 4394.80 | 4 |
| 144 | 227-131 | gctgTCACAcccG | 215 | 227 | 4424.42 | 4 |
| 145 | 234-13H | ggcTACTGCuguC | 222 | 234 | 4425.96 | 6 |
| 146 | 266-131 | gcaaTGCTCccuG | 254 | 266 | 4425.83 | 7 |
| 147 | 273-13H | ggcTGCTGCaauG | 261 | 273 | 4477.55 | 9 |
| 148 | 275-13G | guggCTGCTGcaA | 263 | 275 | 4478.31 | 11 |
| 149 | 275-131 | guggCTGCTgcaA | 263 | 275 | 4507.93 | 11 |
| 150 | 277-13G | caguGGCTGCtgC | 265 | 277 | 4467.81 | 12 |
| 151 | 42-15E | tcccTCCTTGGccuT | 28 | 42 | 5015.14 | 34 |
| 152 | 75-15E | acccTGTTTGGutuT | 61 | 75 | 5077.85 | 35 |
| 153 | 229-15E | cugcTGTCACAcccG | 215 | 229 | 5052.60 | 36 |
| 154 | 254-15E | gctcCCTCCACuguC | 240 | 254 | 4975.31 | 37 |
| 155 | 255-15E | tgcuCCCTCCActgT | 241 | 255 | 5004.69 | 38 |
| 156 | 269-15E | gctgCAATGCTcccT | 255 | 269 | 5066.51 | 40 |
| 157 | 274-15E | tggcTGCTGCAatgC | 260 | 274 | 5147.92 | 41 |
| 158 | 278-15E | ccagTGGCTGCugcA | 264 | 278 | 5120.82 | 42 |
| 159 | 285-15E | gacaAAGCCAGuggC | 271 | 285 | 5176.42 | 43 |
| 160 | 286-15E | tgacAAAGCCAgtgG | 272 | 286 | 5177.30 | 44 |
| 161 | 367-15E | catuGTCAGGAuccA | 353 | 367 | 5104.79 | 47 |
| 162 | 413-15E | ggtuCGTAGTCutgA | 399 | 413 | 5136.87 | 48 |
| 163 | 414-15E | agguTCGTAGTctuG | 400 | 414 | 5137.14 | 49 |
| 164 | 415-15E | caggTTCGTAGucuT | 401 | 415 | 5127.59 | 50 |

In the sequences in Table 10, upper case letters represent DNA, lower case letters represent 2'-OMe-RNA, underlined upper case letters represent 2'-O,4'-C-ethylene nucleoside, and underlined lower case letters represent AmNA. The base part of C in 2'-O,4'-C-ethylene nucleoside and AmNA is 5-methylcytosine. Each nucleoside was bound through phosphorothioate. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 165: Synthesis of HO-G^{e2s}-C^{m1P}-T^{e2p}-G^{s}-T^{s}-5meC^{s}-A^{s}-5meC^{s}-A^{s}-5meC^{s}-C^{e2p}-C^{m1p}-G^{e2t}-H (227-13A1) (SEQ ID NO: 4)

The target compound shown in Table 11 was obtained by synthesis and purification in a similar condition to Example 1. Oxidizer 0.05 M for AKTA (mixed solution of iodine/pyridine/water, manufactured by Sigma-Aldrich, product No. L560250-04) was used as a reagent for forming phosphodiester bond. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 2.508 minutes. The compound was identified by negative ion ESI mass spectrometric analysis (calculated value: 4305.63, measured value: 4305.64).

The base sequence of the compound is complementary to nucleotide number 215 to 227 in the coding region of Homo sapiens synuclein, alpha (SNCA), transcript variant 1, mRNA (NCBI-GenBank accession No.NM_000345).

### Examples 166 to 174

The compounds of Examples 166 to 174 shown in Table 11 were synthesized similarly to Example 165.

**Table 11**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 165 | 227-13A1 | GcoToGTCACACCocoG | 215 | 227 | 4305.64 | 4 |
| 166 | 227-13A2 | GocoToGTCACACCocoG | 215 | 227 | 4289.66 | 4 |
| 167 | 227-13B1 | GcoTogoTCACACCcG | 215 | 227 | 4351.62 | 4 |
| 168 | 227-13B2 | GcoTgoTCACACCocG | 215 | 227 | 4351.62 | 4 |
| 169 | 227-13B3 | GcoTogTCACACCcG | 215 | 227 | 4367.60 | 4 |
| 170 | 227-13B4 | GcoTgoTCACACCcG | 215 | 227 | 4367.59 | 4 |
| 171 | 227-13B5 | GcTogoTCACACCcG | 215 | 227 | 4367.59 | 4 |
| 172 | 227-13D1 | GcoTgoTCACAcoCocG | 215 | 227 | 4351.63 | 4 |
| 173 | 227-13D2 | GcoTgoTCACAcoCcG | 215 | 227 | 4367.61 | 4 |
| 174 | 227-13D3 | GcTgoTCACAcoCocG | 215 | 227 | 4367.61 | 4 |

In the sequences in Table 11, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Between each nucleoside, "o" represents a phosphodiester bond and no description represents a phosphorothioate bond. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Example 175: Synthesis of HO-C^{e2s}-C^{m1p}-T^{e2p}-5meC^{s}-5meC^{s}-T^{s}-T^{s}-G^{s}-G^{s}-5meC^{s}-C^{e2p}-U^{m1p}-T^{e2t}-H (40-13A1) (SEQ ID NO: 2)

The target compound shown in Table 12 was obtained by synthesis and purification in a similar condition to Example 165. The compound was analyzed by reversed phase HPLC (column (Phenomenex, Clarity 2.6 µm Oligo-MS 100A (2.1 × 50 mm)), A solution: 100 mM hexafluoropropanol (HFIP) and 8 mM triethylamine aqueous solution, B solution: methanol, percentage of B: 10% → 25% (4 min, linear gradient); 60°C; 0.5 mL/min; 260 nm). As a result, the compound was eluted at 1.999 minutes. The compound was identified by negative ion ESI mass spectrometric analysis (calculated value: 4262.60, measured value: 4262.61).

The base sequence of the compound is complementary to nucleotide number 28 to 40 in the coding region of Homo sapiens synuclein, alpha (SNCA), transcript variant 1, mRNA (NCBI-GenBank accession No.NM_000345).

### Examples 176 to 197

The compounds of Examples 176 to 197 shown in Table 12 were synthesized similarly to Example 175.

**Table 12**

| Example | Sequence name | Sequence | Target region in hSNCA gene | | Molecular weight (measured value) | Sequence number |
|---|---|---|---|---|---|---|
| | | (5'-3') | 5' end position | 3' end position | | |
| 175 | 40-13A1 | CcoToCCTTGGCCouoT | 28 | 40 | 4262.61 | 2 |
| 176 | 215-13A1 | GuoCoACCACTGCouoC | 203 | 215 | 4280.62 | 3 |
| 177 | 229-13A1 | CuoGoCTGTCACAocoC | 217 | 229 | 4280.63 | 5 |
| 178 | 234-13A1 | GgoCoTACTGCTGouoC | 222 | 234 | 4337.61 | 6 |
| 179 | 266-13A1 | GcoAoATGCTCCCouoG | 254 | 266 | 4306.62 | 7 |
| 180 | 267-13A1 | TgoCoAATGCTCCocoT | 255 | 267 | 4295.62 | 8 |
| 181 | 273-13A1 | GgoCoTGCTGCAAouoG | 261 | 273 | 4372.62 | 9 |
| 182 | 274-13A1 | TgoGoCTGCTGCAoaoT | 262 | 274 | 4361.62 | 10 |
| 183 | 275-13A1 | GuoGoGCTGCTGCoaoA | 263 | 275 | 4372.60 | 11 |
| 184 | 277-13A1 | CaoGoTGGCTGCTogoC | 265 | 277 | 4376.63 | 12 |
| 185 | 412-13A1 | GuoToCGTAGTCTouoG | 400 | 412 | 4325.56 | 13 |
| 186 | 275-13B1 | GuoGogoCTGCTGCaA | 263 | 275 | 4418.59 | 11 |
| 187 | 275-13B3 | GuoGogCTGCTGCaA | 263 | 275 | 4434.58 | 11 |
| 188 | 275-13B4 | GuoGgoCTGCTGCaA | 263 | 275 | 4434.57 | 11 |
| 189 | 275-13B5 | GuGogoCTGCTGCaA | 263 | 275 | 4434.58 | 11 |
| 190 | 277-13B1 | CaoGouoGGCTGCTgC | 265 | 277 | 4408.60 | 12 |
| 191 | 277-13B3 | CaoGouGGCTGCTgC | 265 | 277 | 4424.57 | 12 |
| 192 | 277-13B4 | CaoGuoGGCTGCTgC | 265 | 277 | 4424.58 | 12 |
| 193 | 277-13B5 | CaGouoGGCTGCTgC | 265 | 277 | 4424.58 | 12 |
| 194 | 266-13D1 | GcoAaoTGCTCcoCouG | 254 | 266 | 4352.62 | 7 |
| 195 | 266-13D2 | GcoAaoTGCTCcoCuG | 254 | 266 | 4368.60 | 7 |
| 196 | 275-13D1 | GuoGgoCTGCTgoCoaA | 263 | 275 | 4432.63 | 11 |
| 197 | 275-13D2 | GuoGgoCTGCTgoCaA | 263 | 275 | 4448.61 | 11 |

In the sequences in Table 12, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Between each nucleoside, "o" represents a phosphodiester bond and no description represents a phosphorothioate bond. As the target region, the nucleotide numbers in the coding region of Homo sapiens synuclein, alpha (SNCA), transcript variant 1, mRNA (NCBI-GenBank accession No. NM_000345) are shown. The molecular weight value is measured by negative ion ESI mass spectrometric analysis.

### Examples 198 to 295

The compounds shown in Tables 13 to 19 were synthesized similarly to Example 165.

**Table 13**

| Example | Sequence name | Sequence | Target region in hSNCA gene | | Sequence number |
|---|---|---|---|---|---|
| | | (5'-3') | 5' end position) | 13' end position | |
| 198 | 42-15A1 | TcoCcoTCCTTGGcCuT | 28 | 42 | 34 |
| 199 | 75-15A1 | AcoCcoTGTTTGGuTuT | 61 | 75 | 35 |
| 200 | 229-15A1 | CuoGcoTGTCACAcCcG | 215 | 229 | 36 |
| 201 | 254-15A1 | GcoTcoCCTCCACuGuC | 240 | 254 | 37 |
| 202 | 255-15A1 | TgoCuoCCCTCCAcTgT | 241 | 255 | 38 |
| 203 | 263-15A1 | AuoGcoTCCCTGCuCcC | 249 | 263 | 39 |
| 204 | 269-15A1 | GcoTgoCAATGCTcCcT | 255 | 269 | 40 |
| 205 | 274-15A1 | TgoGcoTGCTGCAaTgC | 260 | 274 | 41 |
| 206 | 278-15A1 | CcoAgoTGGCTGCuGcA | 264 | 278 | 42 |
| 207 | 285-15A1 | GaoCaoAAGCCAGuGgC | 271 | 285 | 43 |
| 208 | 286-15A1 | TgoAcoAAAGCCAgTgG | 272 | 286 | 44 |
| 209 | 288-15A1 | TuoTgoACAAAGCcAgT | 274 | 288 | 45 |
| 210 | 289-15A1 | TuoTuoGACAAAGcCaG | 275 | 289 | 46 |
| 211 | 367-15A1 | CaoTuoGTCAGGAuCcA | 353 | 367 | 47 |
| 212 | 413-15A1 | GgoTuoCGTAGTCuTgA | 399 | 413 | 48 |
| 213 | 414-15A1 | AgoGuoTCGTAGTcTuG | 400 | 414 | 49 |
| 214 | 415-15A1 | CaoGgoTTCGTAGuCuT | 401 | 415 | 50 |

In the sequences in Table 13, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Between each nucleoside, "o" represents a phosphodiester bond and no description represents a phosphorothioate bond. As the target region, the nucleotide numbers in the coding region of Homo sapiens synuclein, alpha (SNCA), transcript variant 1, mRNA (NCBI-GenBank accession No. NM_000345) are shown.

**Table 14**

| Example | Sequence name | Sequence | Target region in hSNCA gene | | Sequence number |
|---|---|---|---|---|---|
| | | (5'-3') | 5' end position | 3' end position | |
| 215 | 42-15B1 | TcoCcoToCCTTGGcCuT | 28 | 42 | 34 |
| 216 | 75-15B1 | AcoCcoToGTTTGGuTuT | 61 | 75 | 35 |
| 217 | 229-15B1 | CuoGcoToGTCACAcCcG | 215 | 229 | 36 |
| 218 | 254-15B1 | GcoTcoCoCTCCACuGuC | 240 | 254 | 37 |
| 219 | 255-15B1 | TgoCuoCoCCTCCAcTgT | 241 | 255 | 38 |
| 220 | 263-15B1 | AuoGcoToCCCTGCuCcC | 249 | 263 | 39 |
| 221 | 269-15B1 | GcoTgoCoAATGCTcCcT | 255 | 269 | 40 |
| 222 | 274-15B1 | TgoGcoToGCTGCAaTgC | 260 | 274 | 41 |
| 223 | 278-15B1 | CcoAgoToGGCTGCuGcA | 264 | 278 | 42 |
| 224 | 285-15B1 | GaoCaoAoAGCCAGuGgC | 271 | 285 | 43 |
| 225 | 286-15B1 | TgoAcoAoAAGCCAgTgG | 272 | 286 | 44 |
| 226 | 288-15B1 | TuoTgoAoCAAAGCcAgT | 274 | 288 | 45 |
| 227 | 289-15B1 | TuoTuoGoACAAAGcCaG | 275 | 289 | 46 |
| 228 | 367-15B1 | CaoTuoGoTCAGGAuCcA | 353 | 367 | 47 |
| 229 | 413-15B1 | GgoTuoCoGTAGTCuTgA | 399 | 413 | 48 |
| 230 | 414-15B1 | AgoGuoToCGTAGTcTuG | 400 | 414 | 49 |
| 231 | 415-15B1 | CaoGgoToTCGTAGuCuT | 401 | 415 | 50 |

In the sequences in Table 14, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Between each nucleoside, "o" represents a phosphodiester bond and no description represents a phosphorothioate bond. As the target region, the nucleotide numbers in the coding region of Homo sapiens synuclein, alpha (SNCA), transcript variant 1, mRNA (NCBI-GenBank accession No. NM_000345) are shown.

**Table 15**

| Example | Sequence name | Sequence | Target region in hSNCA gene | | Sequence number |
|---|---|---|---|---|---|
| | | (5'-3') | 5' end position | 3' end position | |
| 232 | 42-15C1 | TcoCcoTCCTTGGcCuT | 28 | 42 | 34 |
| 233 | 75-15C1 | AcoCcoTGTTTGGuTuT | 61 | 75 | 35 |
| 234 | 229-15C1 | CuoGcoTGTCACAcCcG | 215 | 229 | 36 |
| 235 | 254-15C1 | GcoTcoCCTCCACuGuC | 240 | 254 | 37 |
| 236 | 255-15C1 | TgoCuoCCCTCCAcTgT | 241 | 255 | 38 |
| 237 | 263-15C1 | AuoGcoTCCCTGCuCcC | 249 | 263 | 39 |
| 238 | 269-15C1 | GcoTgoCAATGCTcCcT | 255 | 269 | 40 |
| 239 | 274-15C1 | TgoGcoTGCTGCAaTgC | 260 | 274 | 41 |
| 240 | 278-15C1 | CcoAgoTGGCTGCuGcA | 264 | 278 | 42 |
| 241 | 285-15C1 | GaoCaoAAGCCAGuGgC | 271 | 285 | 43 |
| 242 | 286-15C1 | TgoAcoAAAGCCAgTgG | 272 | 286 | 44 |
| 243 | 288-15C1 | TuoTgoACAAAGCcAgT | 274 | 288 | 45 |
| 244 | 289-15C1 | TuoTuoGACAAAGcCaG | 275 | 289 | 46 |
| 245 | 367-15C1 | CaoTuoGTCAGGAuCcA | 353 | 367 | 47 |
| 246 | 413-15C1 | GgoTuoCGTAGTCuTgA | 399 | 413 | 48 |
| 247 | 414-15C1 | AgoGuoTCGTAGTcTuG | 400 | 414 | 49 |
| 248 | 415-15C1 | CaoGgoTTCGTAGuCuT | 401 | 415 | 50 |

In the sequences in Table 15, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Between each nucleoside, "o" represents a phosphodiester bond and no description represents a phosphorothioate bond. As the target region, the nucleotide numbers in the coding region of Homo sapiens synuclein, alpha (SNCA), transcript variant 1, mRNA (NCBI-GenBank accession No. NM_000345) are shown.

**Table 16**

| Example | Sequence name | Sequence | Target region in hSNCA gene | | Sequence number |
|---|---|---|---|---|---|
| | | (5'-3') | 5' end position | 3' end position | |
| 249 | 42-15D1 | TcoCcoToCCTTGGcCuT | 28 | 42 | 34 |
| 250 | 75-15D1 | AcoCcoToGTTTGGuTuT | 61 | 75 | 35 |
| 251 | 229-15D1 | CuoGcoToGTCACAcCcG | 215 | 229 | 36 |
| 252 | 254-15D1 | GcoTcoCoCTCCACuGuC | 240 | 254 | 37 |
| 253 | 255-15D1 | TgoCuoCoCCTCCAcTgT | 241 | 255 | 38 |
| 254 | 263-15D1 | AuoGcoToCCCTGCuCcC | 249 | 263 | 39 |
| 255 | 269-15D1 | GcoTgoCoAATGCTcCcT | 255 | 269 | 40 |
| 256 | 274-15D1 | TgoGcoToGCTGCAaTgC | 260 | 274 | 41 |
| 257 | 278-15D1 | CcoAgoToGGCTGCuGcA | 264 | 278 | 42 |
| 258 | 285-15D1 | GaoCaoAoAGCCAGuGgC | 271 | 285 | 43 |
| 259 | 286-15D1 | TgoAcoAoAAGCCAgTgG | 272 | 286 | 44 |
| 260 | 288-15D1 | TuoTgoAoCAAAGCcAgT | 274 | 288 | 45 |
| 261 | 289-15D1 | TuoTuoGoACAAAGcCaG | 275 | 289 | 46 |
| 262 | 367-15D1 | CaoTuoGoTCAGGAuCcA | 353 | 367 | 47 |
| 263 | 413-15D1 | GgoTuoCoGTAGTCuTgA | 399 | 413 | 48 |
| 264 | 414-15D1 | AgoGuoToCGTAGTcTuG | 400 | 414 | 49 |
| 265 | 415-15D1 | CaoGgoToTCGTAGuCuT | 401 | 415 | 50 |

In the sequences in Table 16, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Between each nucleoside, "o" represents a phosphodiester bond and no description represents a phosphorothioate bond. As the target region, the nucleotide numbers in the coding region of Homo sapiens synuclein, alpha (SNCA), transcript variant 1, mRNA (NCBI-GenBank accession No. NM_000345) are shown.

**Table 17**

| Example | Sequence name | Sequence | Target region in hSNCA gene | | Sequence number |
|---|---|---|---|---|---|
| | | (5'-3') | 5' end position | 3' end position | |
| 266 | 41-14A1 | CcoCuoCCTTGGcCuT | 28 | 41 | 14 |
| 267 | 42-14A1 | TcoCcoTCCTTGgCcT | 29 | 42 | 15 |
| 268 | 74-14A1 | CcoCuoGTTTGGuTuT | 61 | 74 | 16 |
| 269 | 75-14A1 | AcoCcoTGTTTGgTuT | 62 | 75 | 17 |
| 270 | 228-14A1 | TgoCuoGTCACAcCcG | 215 | 228 | 18 |
| 271 | 229-14A1 | CuoGcoTGTCACaCcC | 216 | 229 | 19 |
| 272 | 266-14A1 | GcoAaoTGCTCCcTgC | 253 | 266 | 20 |
| 273 | 267-14A1 | TgoCaoATGCTCcCuG | 254 | 267 | 21 |
| 274 | 268-14A1 | CuoGcoAATGCTcCcT | 255 | 268 | 22 |
| 275 | 269-14A1 | GcoTgoCAATGCuCcC | 256 | 269 | 23 |
| 276 | 274-14A1 | TgoGcoTGCTGCaAuG | 261 | 274 | 24 |
| 277 | 275-14A1 | GuoGgoCTGCTGcAaT | 262 | 275 | 25 |
| 278 | 284-14A1 | AcoAaoAGCCAGuGgC | 271 | 284 | 26 |
| 279 | 285-14A1 | GaoCaoAAGCCAgTgG | 272 | 285 | 27 |
| 280 | 286-14A1 | TgoAcoAAAGCCaGuG | 273 | 286 | 28 |
| 281 | 366-14A1 | AuoTgoTCAGGAuCcA | 353 | 366 | 29 |
| 282 | 367-14A1 | CaoTuoGTCAGGaTcC | 354 | 367 | 30 |
| 283 | 412-14A1 | GuoTcoGTAGTCuTgA | 399 | 412 | 31 |
| 284 | 413-14A1 | GgoTuoCGTAGTcTuG | 400 | 413 | 32 |
| 285 | 414-14A1 | AgoGuoTCGTAGuCuT | 401 | 414 | 33 |

In the sequences in Table 17, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Between each nucleoside, "o" represents a phosphodiester bond and no description represents a phosphorothioate bond. As the target region, the nucleotide numbers in the coding region of Homo sapiens synuclein, alpha (SNCA), transcript variant 1, mRNA (NCBI-GenBank accession No. NM_000345) are shown.

**Table 18**

| Example | Sequence name | Sequence | Target region in hSNCA gene | | Sequence number |
|---|---|---|---|---|---|
| | | (5'-3') | 5' end position 3' end position | | |
| 286 | 43-16A1 | CuoCcoCTCCTTGGcCuT | 28 | 43 | 51 |
| 287 | 76-16A1 | CaoCcoCTGTTTGGuTuT | 61 | 76 | 52 |
| 288 | 230-16A1 | AcoTgoCTGTCACAcCcG | 215 | 230 | 53 |
| 289 | 255-16A1 | TgoCuoCCCTCCACuGuC | 240 | 255 | 54 |
| 290 | 269-16A1 | GcoTgoCAATGCTCcCuG | 254 | 269 | 55 |
| 291 | 278-16A1 | CcoAgoTGGCTGCTgCaA | 263 | 278 | 56 |
| 292 | 286-16A1 | TgoAcoAAAGCCAGuGgC | 271 | 286 | 57 |
| 293 | 368-16A1 | TcoAuoTGTCAGGAuCcA | 353 | 368 | 58 |
| 294 | 414-16A1 | AgoGuoTCGTAGTCuTgA | 399 | 414 | 59 |

In the sequences in Table 18, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Between each nucleoside, "o" represents a phosphodiester bond and no description represents a phosphorothioate bond. As the target region, the nucleotide numbers in the coding region of Homo sapiens synuclein, alpha (SNCA), transcript variant 1, mRNA (NCBI-GenBank accession No. NM_000345) are shown.

**Table 19**

| Example | Sequence name | Sequence | Target region in hSNCA gene | | Sequence number |
|---|---|---|---|---|---|
| | | (5'-3') | 5' end position | 3' end position | |
| 295 | 227-13C | GcTGTCACAcCcG | 215 | 227 | 4 |

In the sequences in Table 19, upper case letters represent DNA, lower case letters represent 2'-OMe-RNA, and underlined upper case letters represent 2'-O,4'-C-ethylene nucleoside. The base part of C in DNA and 2'-O,4'-C-ethylene nucleoside is 5-methylcytosine. Between each nucleoside, "o" represents a phosphodiester bond and no description represents a phosphorothioate bond. As the target region, the nucleotide numbers in the coding region of Homo sapiens synuclein, alpha (SNCA), transcript variant 1, mRNA (NCBI-GenBank accession No. NM_000345) are shown.

In this disclosure, A^{t}, G^{t}, 5meC^{t}, C^{t}, T^{t}, U^{t}, A^{p}, G^{p}, 5meC^{p}, C^{p}, T^{p}, U^{p}, A^{s}, G^{s}, 5meC^{s}, C^{s}, T^{s}, U^{s}, A^{m1t}, G^{m1t}, C^{m1t}, 5meC^{m1t}, U^{m1t}, A^{m1p}, G^{m1p}, C^{m1p}, 5meC^{m1p}, U^{m1p}, A^{m1s}, G^{m1s}, C^{m1s}, 5meC^{m1s}, U^{m1s}, A^{2t}, G^{2t}, C^{2t}, T^{2t}, A^{e2p}, G^{e2p}, C^{e2p}, T^{e2p}, A^{e2s}, G^{e2s}, C^{e2s}, T^{e2s}, A^{1t}, G^{1t}, C^{1t}, T^{1t}, A^{e1p}, G^{e1p}, C^{e1p}, T^{e1p}, A^{e1s}, G^{e1s}, C^{e1s}, T^{e1s}, A^{3t}, G^{3t}, C^{3t}, T^{3t}, A^{e3p}, G^{e3p}, C^{e3p}, T^{e3p}, A^{e3s}, G^{e3s}, C^{e3s}, T^{e3s}, A^{m2t}, G^{m2t}, 5meC^{m2t}, T^{m2t}, A^{m2p}, G^{m2p}, 5meC^{m2p}, T^{m2p}, A^{m2s}, G^{m2s}, 5meC^{m2s} and T^{m2s} are groups having the following structures.

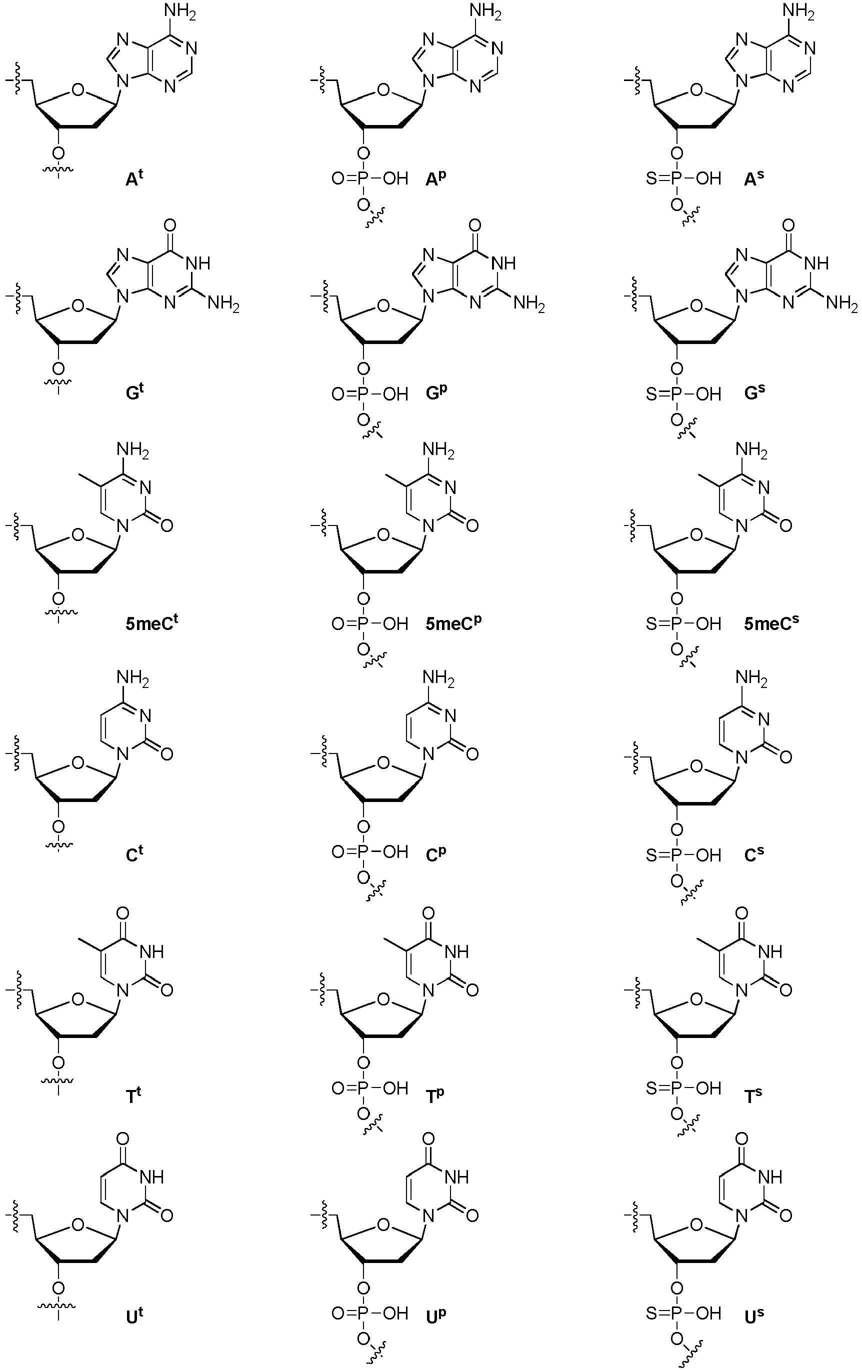

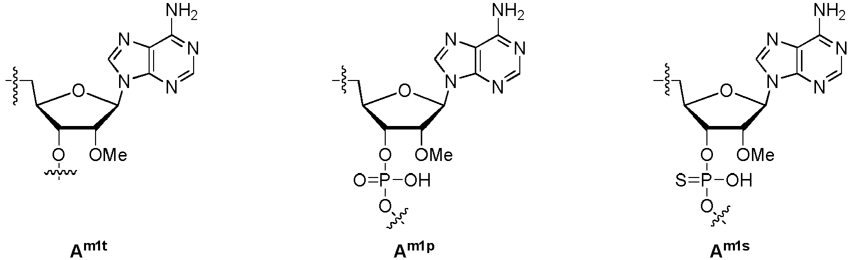

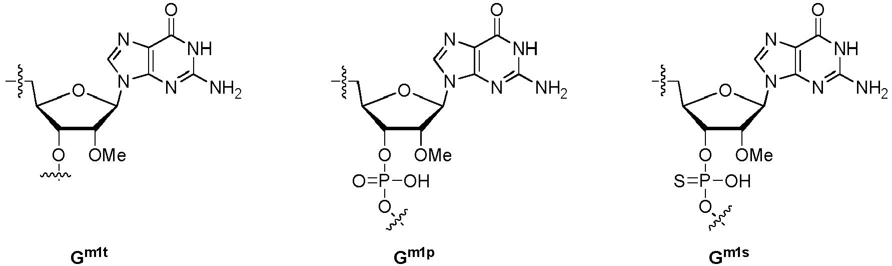

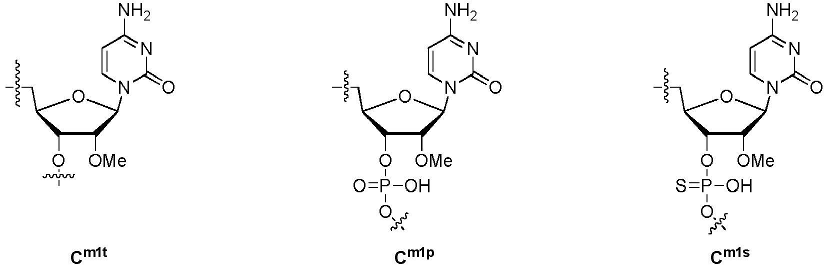

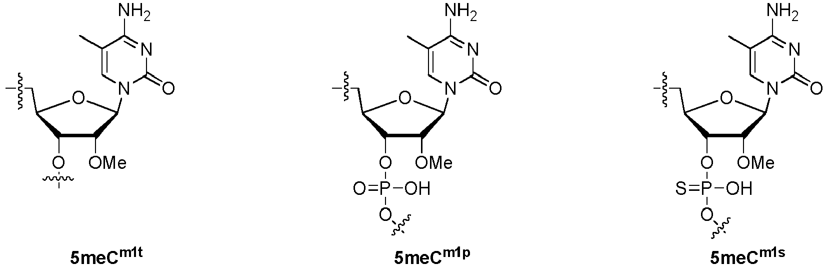

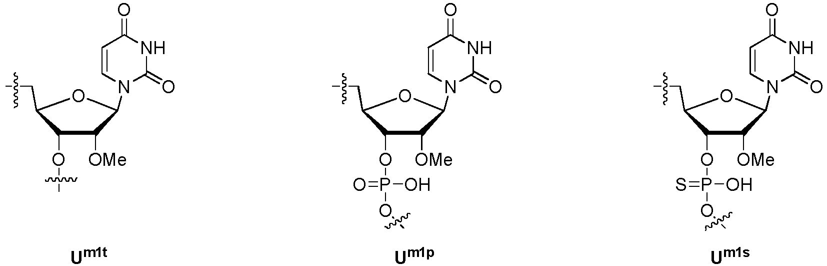

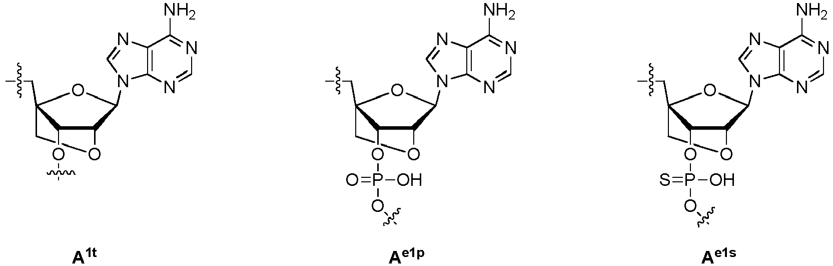

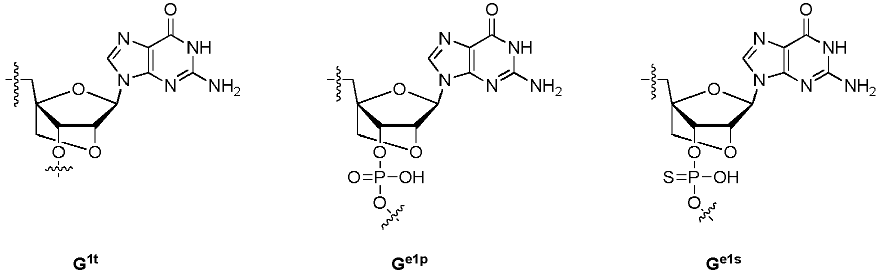

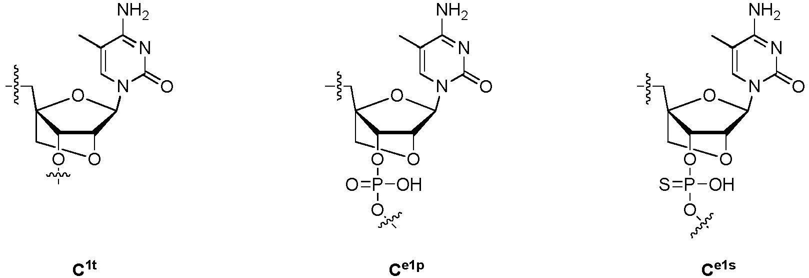

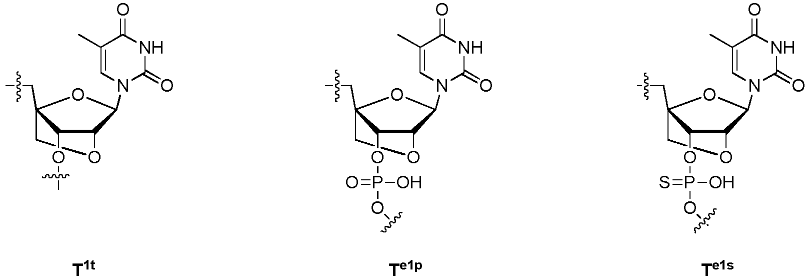

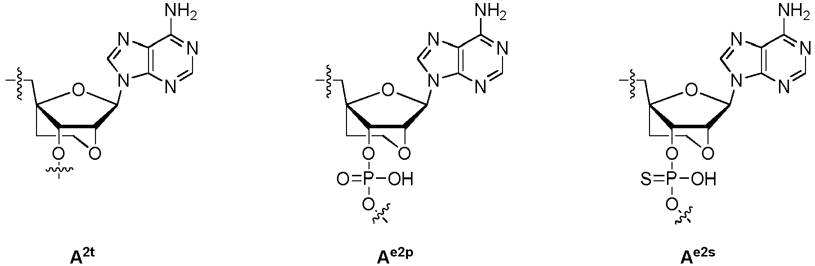

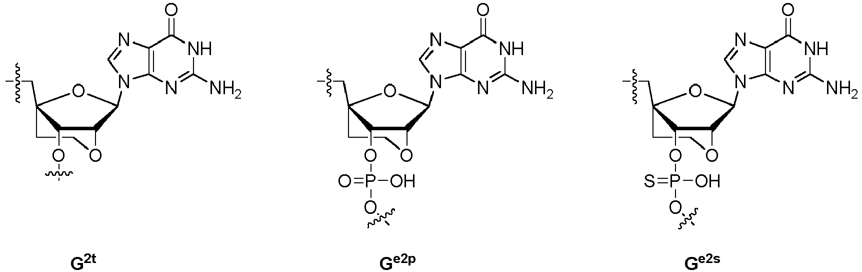

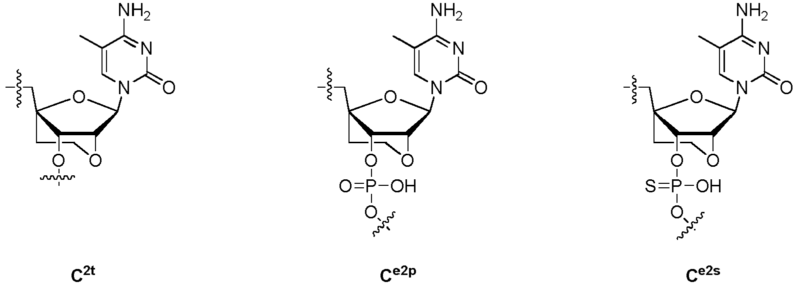

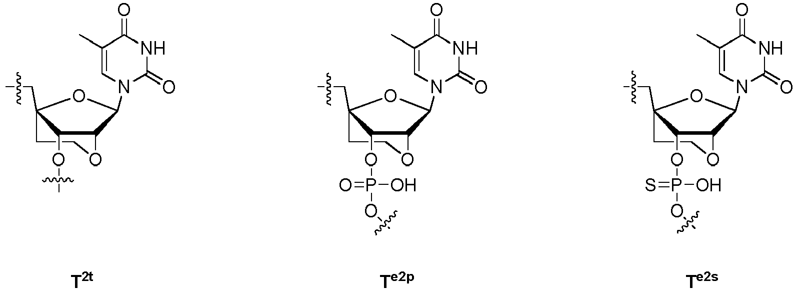

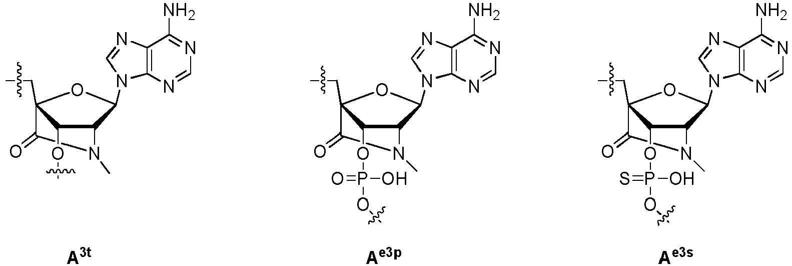

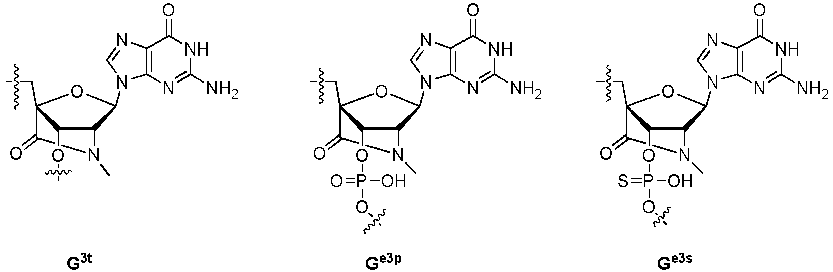

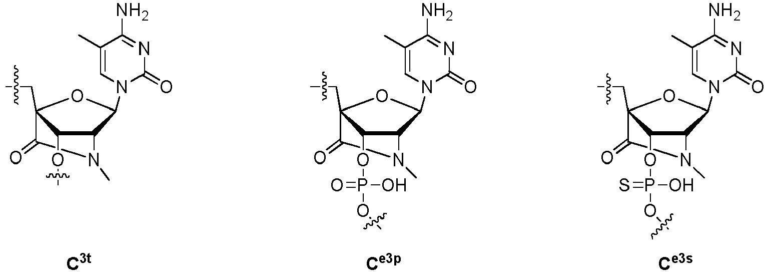

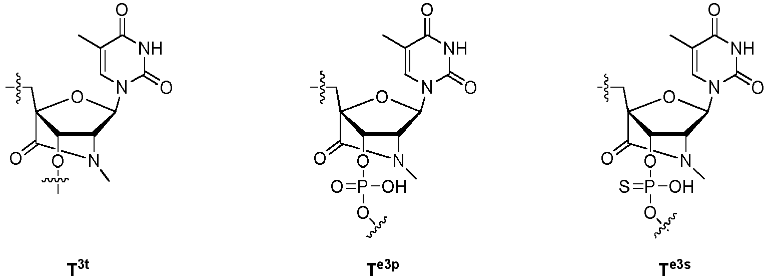

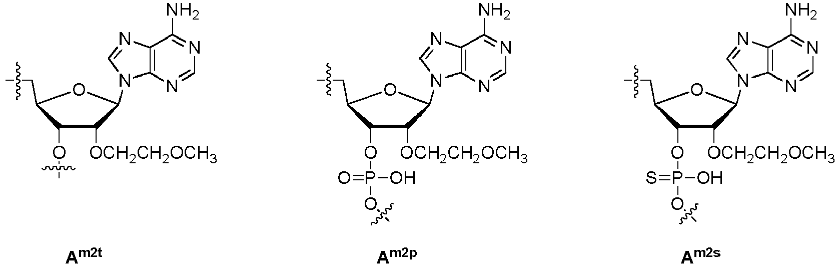

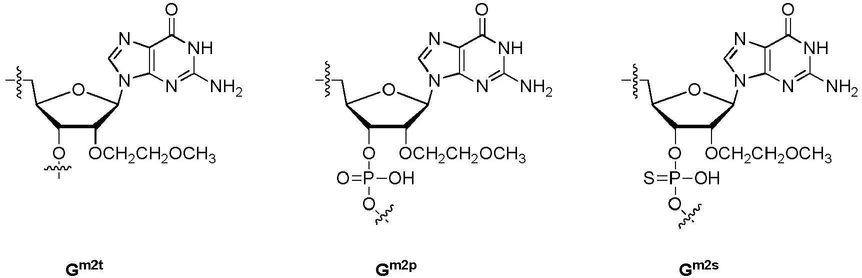

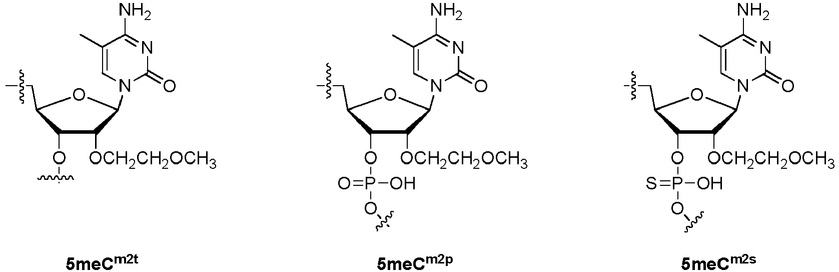

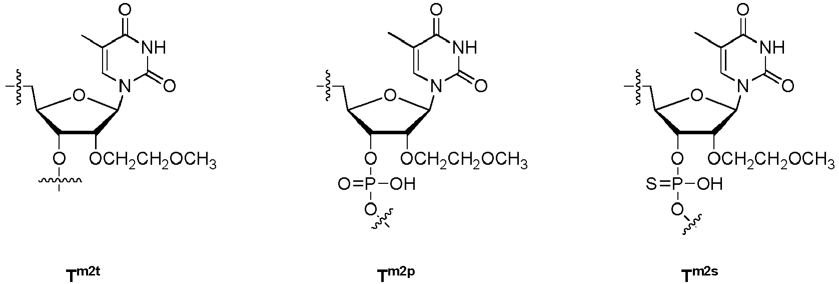

### Test example 1: Additional screening based on transfection of ASO into HEK293A cell (1)

The day before the transfection, 5 × 10⁴ of HEK293A cell ("model number: R705-07" manufactured by ATCC) was inoculated in 0.5 mL of DMEM medium (manufactured by Thermo Fisher Scientific) containing 10% FBS (manufactured by HyClone) on 24 well plate (manufactured by Thermo Fisher Scientific). Two well, specifically one well per one kind of ASO and other one well of distilled deionized water ("ddW" manufactured by NACALAI TESQUE) as a control which did not contain ASO, were prepared.

On one well of 96 well plate (manufactured by Applied Biosystems), 2.5 µL of ASO having a concentration of 10 µM was added to 35 µL of Opti-MEM (manufactured by Thermo Fisher Scientific), and 37.5 µL of OptiMEM and 0.9 µL of Lipofectamine RNAiMAX (manufactured by Invitrogen) were added thereto. The mixture was left to stand at room temperature for about 20 minutes and then added to the cell. A well of a control which did not contain ASO was prepared as follows. On two wells, 2.5 µL of ddW was added to 35 µL of Opti-MEM per one well, and 37.5 µL of OptiMEM to which 0.9 µL of Lipofectamine RNAiMAX was added was further added thereto per one well. The wells were left to stand at room temperature for 20 minutes, and then the mixture was added to the cell. After about 24 hours, each well was washed with PBS (manufactured by Thermo Fisher Scientific) two times.

RNA extraction and reverse transcription reaction were performed by using SuperPrep Cell Lysis RT Kit for qPCR (manufactured by Takara Bio) in accordance with the protocol attached to the kit. The thus obtained cDNA was diluted with ddW three times, and an amount of mRNA of endogenous human α-synuclein (hSNCA) was measured by quantitative PCR.

Quantitative PCR was performed as follows by using TagMan Gene Expression Assay (manufactured by Applied Biosystems). The obtained 20 µL of cDNA was diluted 5 times with 80 µL of ddW. Per one well of 384 PCR plate (manufactured by Applied Biosystems), 2×Taqman probe master mix, ddW, cDNA diluted 5 times, hSNCA primer ("Hs01103383" manufactured by Applied Biosystems) and β-Actin primer ("Hs99999903" manufactured by Applied Biosystems) were mixed in a ratio of 5 : 2 : 2 : 0.5 : 0.5 (µL) to prepare 10 µL of mixture. The mixture was prepared on two wells per each cDNA. Real-time PCR was performed by using Viia 7 (manufactured by Applied Biosystems) to measure an amount of mRNA of SNCA as an average value of duplicate. Average values of two wells of cDNA into which ddW which did not contain ASO was transfected were averaged, and the averaged value was used as a control.

The above-described experiment was repeated three times, and an average value and a standard deviation (SD) were calculated.

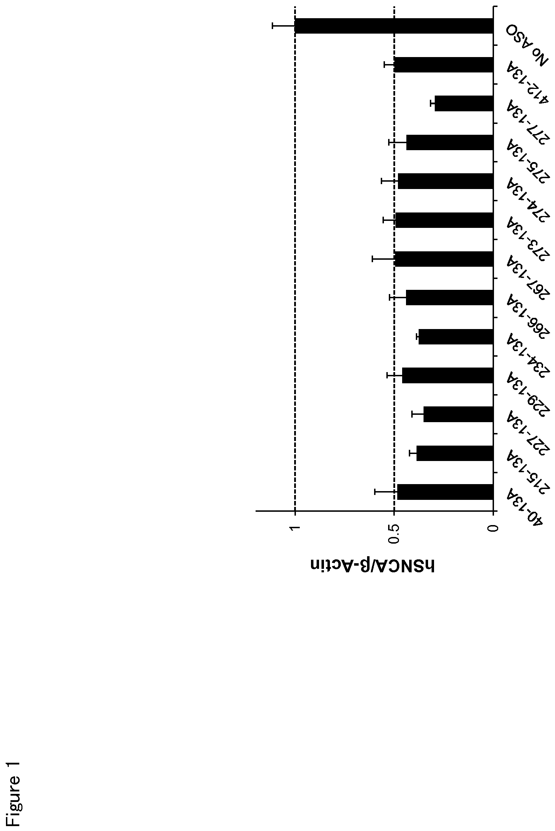

The result is shown in Figure 1. An amount of mRNA is put on the vertical axis of Figure 1. The amounts of mRNA after the transfection are relatively demonstrated on the premise that the amount of mRNA of control (shown as "No ASO" in Figure 1) is 1.0. The ASO of which sequence name was 40-13A, 215-13A, 227-13A, 229-13A, 234-13A, 266-13A, 267-13A, 273-13A, 274-13A, 275-13A, 277-13A and 412-13A exhibited an excellent effect to suppress α-synuclein mRNA.

### Test example 2: Additional screening based on transfection of ASO into HEK293A cell (2)

ASO was transfected into HEK293A cell similarly to Test example 1 to measure an amount of mRNA and calculate an average value and SD of 4 experiments.

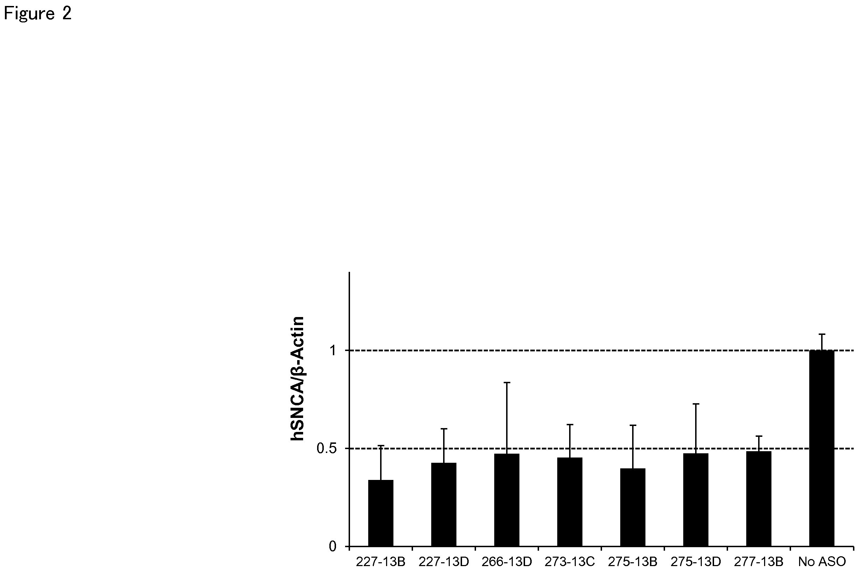

The result is shown in Figure 2. An amount of mRNA is put on the vertical axis of Figure 2. The amounts of mRNA after the transfection are relatively demonstrated on the premise that the amount of mRNA of control (shown as "No ASO" in Figure 2) is 1.0. The ASO of which sequence name was 227-13B, 227-13D, 266-13D, 273-13C, 275-13B, 275-13D and 277-13B exhibited an excellent effect to suppress α-synuclein mRNA.

### Test example 3: Additional screening based on transfection of ASO into HEK293A cell (3)

ASO was transfected into HEK293A cell similarly to Test example 1 to measure an amount of mRNA and calculate an average value and SD of 3 experiments.

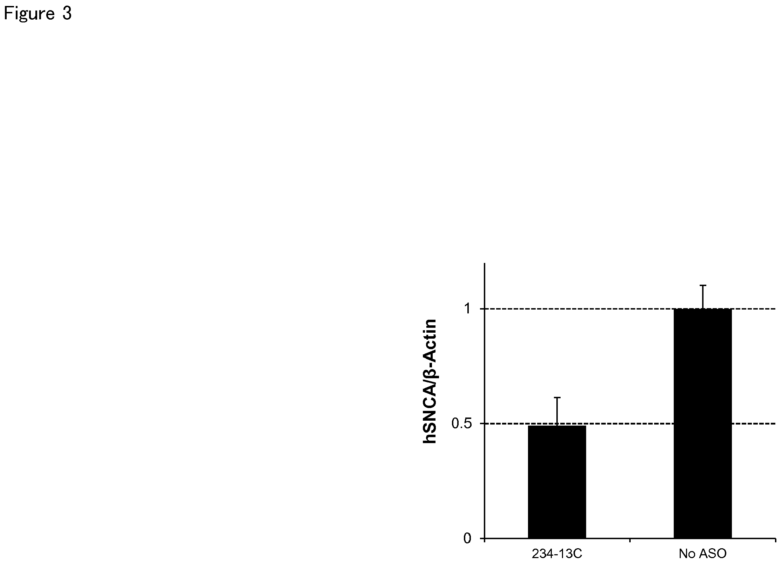

The result is shown in Figure 3. An amount of mRNA is put on the vertical axis of Figure 3. The amounts of mRNA after the transfection are relatively demonstrated on the premise that the amount of mRNA of control (shown as "No ASO" in Figure 3) is 1.0. The ASO of which sequence name was 234-13C exhibited an excellent effect to suppress α-synuclein mRNA.

### Test example 4: Additional screening based on transfection of ASO into HEK293A cell (4)

ASO was transfected into HEK293A cell similarly to Test example 1 to measure an amount of mRNA and calculate an average value and SD of 4 experiments.

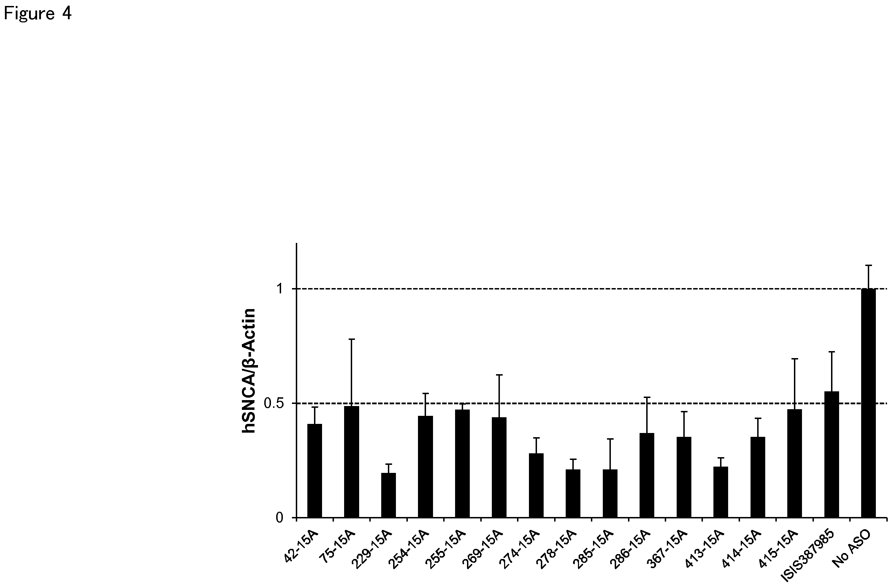

The result is shown in Figure 4. An amount of mRNA is put on the vertical axis of Figure 4. The amounts of mRNA after the transfection are relatively demonstrated on the premise that the amount of mRNA of control (shown as "No ASO" in Figure 3) is 1.0. The ASO of which sequence name was 42-15A, 75-15A, 229-15A, 254-15A, 255-15A, 269-15A, 274-15A, 278-15A, 285-15A, 286-15A, 367-15A, 413-15A, 414-15A and 415-15A exhibited an excellent effect to suppress α-synuclein mRNA. These ASO exhibited superior effect to suppress mRNA in comparison with ISIS387985 described in JP 2014-501507.

### Test example 5: Additional screening based on transfection of ASO into HEK293A cell (5)

ASO was transfected into HEK293A cell similarly to Test example 1 to measure an amount of mRNA and calculate an average value and SD of 4 experiments.

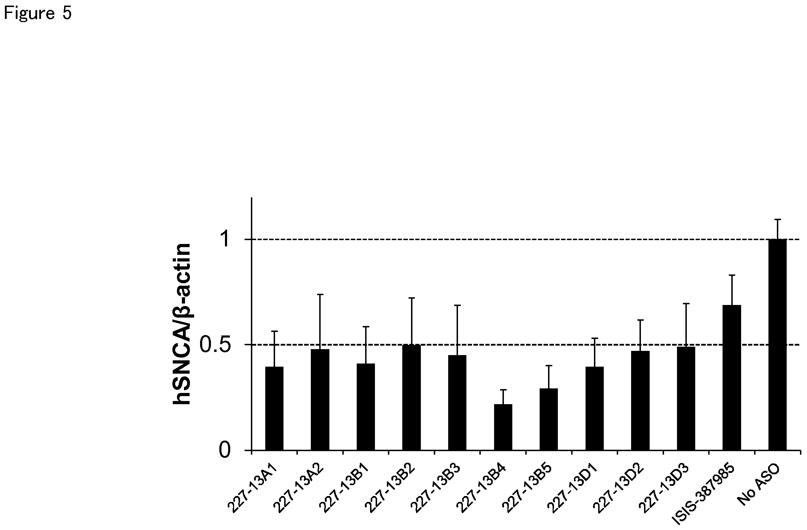

The result is shown in Figure 5. An amount of mRNA is put on the vertical axis of Figure 5. The amounts of mRNA after the transfection are relatively demonstrated on the premise that the amount of mRNA of control (shown as "No ASO" in Figure 3) is 1.0. The ASO of which sequence name was 227-13A1, 227-13A2, 227-13B1, 227-13B2, 227-13B3, 227-13B4, 227-13B5, 227-13D1, 227-13D2 and 227-13D3 exhibited an excellent effect to suppress α-synuclein mRNA. These ASO exhibited superior effect to suppress mRNA in comparison with ISIS387985 described in JP 2014-501507.

### INDUSTRIAL APPLICABILITY

The present invention provides the oligonucleotide which is useful for suppressing an expression of α-synuclein. The oligonucleotide of the present invention can be expected to be utilized as nucleic acid therapeutics useful for treating or preventing α-synuclein excess symptom, Parkinson's disease, Lewy body dementia or the like.

## Claims

1. An oligonucleotide or a pharmacologically acceptable salt thereof,
comprising at least one 2'-O,4'-C-ethylene nucleoside,
wherein the oligonucleotide can hybridize with α-synuclein gene, has an activity to suppress an expression of the α-synuclein gene, and is complementary to the α-synuclein gene, 5' end of the oligonucleotide is a nucleotide complementary to any one nucleotide selected from the group consisting of the 40^{th} to 43^{rd} positions, the 74^{th} to 76^{th} positions, the 215^{th} position, the 227^{th} to 230^{th} positions, the 234^{th} position, the 254^{th} position, the 255^{th} position, the 263^{rd} position, the 266^{th} to 269^{th} positions, the 273^{rd} to 275^{th} positions, the 277^{th} position, the 278^{th} position, the 284^{th} to 286^{th} positions, the 288^{th} position, the 289^{th} position, the 366^{th} to 368^{th} positions, and the 412^{nd} to 415^{th} positions of SEQ ID NO: 1,
the oligonucleotide is complementary to at least a part of SEQ ID NO: 1, and
the oligonucleotide has a length of 13 or more and 16 or less nucleotides.

2. The oligonucleotide or pharmacologically acceptable salt thereof according to claim 1, wherein the 5' end of the oligonucleotide is a nucleotide complementary to any one nucleotide selected from the group consisting of the 40^{th} to 42^{nd} positions, the 74^{th} to 76^{th} positions, the 215^{th} position, the 227^{th} to 230^{th} positions, the 234^{th} position, the 254^{th} position, the 255^{th} position, the 263^{rd} position, the 266^{th} position, the 267^{th} position, the 269^{th} position, the 273^{rd} to 275^{th} positions, the 277^{th} position, the 278^{th} position, the 284^{th} to 286^{th} positions, the 288^{th} position, the 289^{th} position, the 366^{th} to 368^{th} positions, and the 412^{nd} to 415^{th} positions of SEQ ID NO: 1, the oligonucleotide is complementary to at least a part of SEQ ID NO: 1, and the oligonucleotide has a length of 13 or more and 16 or less nucleotides.

3. The oligonucleotide or pharmacologically acceptable salt thereof according to claim 1, wherein the 5' end of the oligonucleotide is a nucleotide complementary to any one nucleotide selected from the group consisting of the 41^{st} position, the 42^{nd} position, the 215^{th} position, the 227^{th} to 230^{th} positions, the 234^{th} position, the 274^{th} position, the 277^{th} position, the 278^{th} position, the 284^{th} to 286^{th} positions, the 288^{th} position, the 366^{th} to 368^{th} positions, and the 412^{nd} to 414^{th} positions of SEQ ID NO: 1, the oligonucleotide is complementary to at least a part of SEQ ID NO: 1, and the oligonucleotide has a length of 13 or more and 16 or less nucleotides.

4. The oligonucleotide or pharmacologically acceptable salt thereof according to claim 1, wherein the 5' end of the oligonucleotide is a nucleotide complementary to any one nucleotide selected from the group consisting of the 42^{nd} position, the 227^{th} to 230^{th} positions, the 274^{th} position, the 277^{th} position, the 278^{th} position, the 284^{th} to 286^{th} positions, the 413^{rd} position, and the 414^{th} position of SEQ ID NO: 1, the oligonucleotide is complementary to at least a part of SEQ ID NO: 1, and the oligonucleotide has a length of 13 or more and 16 or less nucleotides.

5. The oligonucleotide or pharmacologically acceptable salt thereof according to claim 1, wherein the 5' end of the oligonucleotide is a nucleotide complementary to any one nucleotide selected from the group consisting of the 42^{nd} position, the 227^{th} position, the 229^{th} position, the 274^{th} position, the 277^{th} position, the 278^{th} position, the 285^{th} position, and the 413^{rd} position of SEQ ID NO: 1, the oligonucleotide is complementary to at least a part of SEQ ID NO: 1, and the oligonucleotide has a length of 13 or more and 16 or less nucleotides.

6. The oligonucleotide or pharmacologically acceptable salt thereof according to claim 1, wherein the 5' end of the oligonucleotide is a nucleotide complementary to any one nucleotide selected from the group consisting of the 227^{th} position, the 229^{th} position, the 278^{th} position, the 285^{th} position, and the 413^{rd} position of SEQ ID NO: 1, the oligonucleotide is complementary to at least a part of SEQ ID NO: 1, and the oligonucleotide has a length of 13 or more and 16 or less nucleotides.

7. The oligonucleotide or pharmacologically acceptable salt thereof according to claim 1, wherein the 5' end of the oligonucleotide is a nucleotide complementary to any one nucleotide selected from the group consisting of the 229^{th} position, the 278^{th} position, the 285^{th} position, and the 413^{rd} position of SEQ ID NO: 1, the oligonucleotide is complementary to at least a part of SEQ ID NO: 1, and the oligonucleotide has a length of 13 nucleotides.

8. The oligonucleotide or pharmacologically acceptable salt thereof according to any one of claims 1 to 7,
wherein the oligonucleotide is a gapmer consisting of a gap region having a length of 5 or more and 7 or less bases, a 5' wing having a length of 3 or more and 5 or less bases, and a 3' wing having a length of 3 or more and 5 or less bases,
the gap region is placed between the 5' wing and the 3' wing,
the 5' wing and the 3' wing comprise at least one 2'-O,4'-C-ethylene nucleoside, and
the oligonucleotide has a length of 13 or more and 16 or less nucleotides.

9. The oligonucleotide or pharmacologically acceptable salt thereof according to any one of claims 1 to 8, wherein a phosphodiester bond is modified to be a phosphorothioate bond.

10. An α-synuclein expression inhibitor comprising the oligonucleotide or pharmacologically acceptable salt thereof according to any one of claims 1 to 9 as an active ingredient.

11. A pharmaceutical composition comprising the oligonucleotide or pharmacologically acceptable salt thereof according to any one of claims 1 to 9 as an active ingredient.

12. The pharmaceutical composition according to claim 11, used for treating or preventing α-synuclein excess symptom.

13. The pharmaceutical composition according to claim 11, used for treating or preventing Parkinson's disease or Lewy body dementia.


SEQUENCE LISTING

<110>  OSAKA UNIVERSITY
       DAIICHI SANKYO COMPANY, LIMITED

<120>  ENA Antisense oligonucleotide for inhibiting \203¿-synuclein
expression

<130>  F18-072PCT

<150>  JP2017-132290
<151>  2017-07-05

<160>  59

<210>  1
<211>  423
<212>  DNA
<213>  Homo sapiens
<220>
<223>  \203¿-synuclein gene
<400>  1
atg gat gta ttc atg aaa gga ctt tca aag gcc aag gag gga gtt gtg      48
Met Asp Val Phe Met Lys Gly Leu Ser Lys Ala Lys Glu Gly Val Val
1               5                   10                  15

gct gct gct gag aaa acc aaa cag ggt gtg gca gaa gca gca gga aag      96
Ala Ala Ala Glu Lys Thr Lys Gln Gly Val Ala Glu Ala Ala Gly Lys
            20                  25                  30

aca aaa gag ggt gtt ctc tat gta ggc tcc aaa acc aag gag gga gtg      144
Thr Lys Glu Gly Val Leu Tyr Val Gly Ser Lys Thr Lys Glu Gly Val
        35                  40                  45

gtg cat ggt gtg aca aca gtg gct gag aag acc aaa gag caa gtg aca      192
Val His Gly Val Thr Thr Val Ala Glu Lys Thr Lys Glu Gln Val Thr
    50                  55                  60

aat gtt gga gga gca gtg gtg acg ggt gtg aca gca gta gcc cag aag      240
Asn Val Gly Gly Ala Val Val Thr Gly Val Thr Ala Val Ala Gln Lys
65                  70                  75                  80

aca gtg gag gga gca ggg agc att gca gca gcc act ggc ttt gtc aaa      288
Thr Val Glu Gly Ala Gly Ser Ile Ala Ala Ala Thr Gly Phe Val Lys
                85                  90                  95

aag gac cag ttg ggc aag aat gaa gaa gga gcc cca cag gaa gga att      336
Lys Asp Gln Leu Gly Lys Asn Glu Glu Gly Ala Pro Gln Glu Gly Ile
            100                 105                 110

ctg gaa gat atg cct gtg gat cct gac aat gag gct tat gaa atg cct      384
Leu Glu Asp Met Pro Val Asp Pro Asp Asn Glu Ala Tyr Glu Met Pro
        115                 120                 125

tct gag gaa ggg tat caa gac tac gaa cct gaa gcc taa      423
Ser Glu Glu Gly Tyr Gln Asp Tyr Glu Pro Glu Ala
    130                 135                 140

<210>  2
<211>  13
<212>  DNA
<213>  Artificial sequence
<220>

<223>  antisense nucleotide
<400>  2
cctccttggc ctt     13

<210>  3
<211>  13
<212>  DNA
<213>  Artificial sequence
<220>
<223>  antisense nucleotide
<400>  3
gtcaccactg ctc     13

<210>  4
<211>  13
<212>  DNA
<213>  Artificial sequence
<220>
<223>  antisense nucleotide
<400>  4
gctgtcacac ccg     13

<210>  5
<211>  13
<212>  DNA
<213>  Artificial sequence
<220>
<223>  antisense nucleotide
<400>  5
ctgctgtcac acc     13

<210>  6
<211>  13
<212>  DNA
<213>  Artificial sequence
<220>
<223>  antisense nucleotide
<400>  6
ggctactgct gtc     13

<210>  7
<211>  13
<212>  DNA
<213>  Artificial sequence
<220>
<223>  antisense nucleotide
<400>  7
gcaatgctcc ctg     13

<210>  8
<211>  13
<212>  DNA
<213>  Artificial sequence
<220>
<223>  antisense nucleotide
<400>  8
tgcaatgctc cct     13

<210>  9
<211>  13
<212>  DNA
<213>  Artificial sequence
<220>

<223>  antisense nucleotide
<400>  9
ggctgctgca atg     13

<210>  10
<211>  13
<212>  DNA
<213>  Artificial sequence
<220>
<223>  antisense nucleotide
<400>  10
tggctgctgc aat     13

<210>  11
<211>  13
<212>  DNA
<213>  Artificial sequence
<220>
<223>  antisense nucleotide
<400>  11
gtggctgctg caa     13

<210>  12
<211>  13
<212>  DNA
<213>  Artificial sequence
<220>
<223>  antisense nucleotide
<400>  12
cagtggctgc tgc     13

<210>  13
<211>  13
<212>  DNA
<213>  Artificial sequence
<220>
<223>  antisense nucleotide
<400>  13
gttcgtagtc ttg     13

<210>  14
<211>  14
<212>  DNA
<213>  Artificial sequence
<220>
<223>  antisense nucleotide
<400>  14
ccctccttgg cctt     14

<210>  15
<211>  14
<212>  DNA
<213>  Artificial sequence
<220>
<223>  antisense nucleotide
<400>  15
tccctccttg gcct     14

<210>  16
<211>  14
<212>  DNA
<213>  Artificial sequence
<220>

<223>  antisense nucleotide
<400>  16
ccctgtttgg tttt     14

<210>  17
<211>  14
<212>  DNA
<213>  Artificial sequence
<220>
<223>  antisense nucleotide
<400>  17
accctgtttg gttt     14

<210>  18
<211>  14
<212>  DNA
<213>  Artificial sequence
<220>
<223>  antisense nucleotide
<400>  18
tgctgtcaca cccg     14

<210>  19
<211>  14
<212>  DNA
<213>  Artificial sequence
<220>
<223>  antisense nucleotide
<400>  19
ctgctgtcac accc     14

<210>  20
<211>  14
<212>  DNA
<213>  Artificial sequence
<220>
<223>  antisense nucleotide
<400>  20
gcaatgctcc ctgc     14

<210>  21
<211>  14
<212>  DNA
<213>  Artificial sequence
<220>
<223>  antisense nucleotide
<400>  21
tgcaatgctc cctg     14

<210>  22
<211>  14
<212>  DNA
<213>  Artificial sequence
<220>
<223>  antisense nucleotide
<400>  22
ctgcaatgct ccct     14

<210>  23
<211>  14
<212>  DNA
<213>  Artificial sequence
<220>

<223>  antisense nucleotide
<400>  23
gctgcaatgc tccc     14

<210>  24
<211>  14
<212>  DNA
<213>  Artificial sequence
<220>
<223>  antisense nucleotide
<400>  24
tggctgctgc aatg     14

<210>  25
<211>  14
<212>  DNA
<213>  Artificial sequence
<220>
<223>  antisense nucleotide
<400>  25
gtggctgctg caat     14

<210>  26
<211>  14
<212>  DNA
<213>  Artificial sequence
<220>
<223>  antisense nucleotide
<400>  26
acaaagccag tggc     14

<210>  27
<211>  14
<212>  DNA
<213>  Artificial sequence
<220>
<223>  antisense nucleotide
<400>  27
gacaaagcca gtgg     14

<210>  28
<211>  14
<212>  DNA
<213>  Artificial sequence
<220>
<223>  antisense nucleotide
<400>  28
tgacaaagcc agtg     14

<210>  29
<211>  14
<212>  DNA
<213>  Artificial sequence
<220>
<223>  antisense nucleotide
<400>  29
attgtcagga tcca     14

<210>  30
<211>  14
<212>  DNA
<213>  Artificial sequence
<220>

<223>  antisense nucleotide
<400>  30
cattgtcagg atcc     14

<210>  31
<211>  14
<212>  DNA
<213>  Artificial sequence
<220>
<223>  antisense nucleotide
<400>  31
gttcgtagtc ttga     14

<210>  32
<211>  14
<212>  DNA
<213>  Artificial sequence
<220>
<223>  antisense nucleotide
<400>  32
ggttcgtagt cttg     14

<210>  33
<211>  14
<212>  DNA
<213>  Artificial sequence
<220>
<223>  antisense nucleotide
<400>  33
aggttcgtag tctt     14

<210>  34
<211>  15
<212>  DNA
<213>  Artificial sequence
<220>
<223>  antisense nucleotide
<400>  34
tccctccttg gcctt     15

<210>  35
<211>  15
<212>  DNA
<213>  Artificial sequence
<220>
<223>  antisense nucleotide
<400>  35
accctgtttg gtttt     15

<210>  36
<211>  15
<212>  DNA
<213>  Artificial sequence
<220>
<223>  antisense nucleotide
<400>  36
ctgctgtcac acccg     15

<210>  37
<211>  15
<212>  DNA
<213>  Artificial sequence
<220>

<223>  antisense nucleotide
<400>  37
gctccctcca ctgtc     15

<210>  38
<211>  15
<212>  DNA
<213>  Artificial sequence
<220>
<223>  antisense nucleotide
<400>  38
tgctccctcc actgt     15

<210>  39
<211>  15
<212>  DNA
<213>  Artificial sequence
<220>
<223>  antisense nucleotide
<400>  39
atgctccctg ctccc     15

<210>  40
<211>  15
<212>  DNA
<213>  Artificial sequence
<220>
<223>  antisense nucleotide
<400>  40
gctgcaatgc tccct     15

<210>  41
<211>  15
<212>  DNA
<213>  Artificial sequence
<220>
<223>  antisense nucleotide
<400>  41
tggctgctgc aatgc     15

<210>  42
<211>  15
<212>  DNA
<213>  Artificial sequence
<220>
<223>  antisense nucleotide
<400>  42
ccagtggctg ctgca     15

<210>  43
<211>  15
<212>  DNA
<213>  Artificial sequence
<220>
<223>  antisense nucleotide
<400>  43
gacaaagcca gtggc     15

<210>  44
<211>  15
<212>  DNA
<213>  Artificial sequence
<220>

<223>  antisense nucleotide
<400>  44
tgacaaagcc agtgg     15

<210>  45
<211>  15
<212>  DNA
<213>  Artificial sequence
<220>
<223>  antisense nucleotide
<400>  45
tttgacaaag ccagt     15

<210>  46
<211>  15
<212>  DNA
<213>  Artificial sequence
<220>
<223>  antisense nucleotide
<400>  46
ttttgacaaa gccag     15

<210>  47
<211>  15
<212>  DNA
<213>  Artificial sequence
<220>
<223>  antisense nucleotide
<400>  47
cattgtcagg atcca     15

<210>  48
<211>  15
<212>  DNA
<213>  Artificial sequence
<220>
<223>  antisense nucleotide
<400>  48
ggttcgtagt cttga     15

<210>  49
<211>  15
<212>  DNA
<213>  Artificial sequence
<220>
<223>  antisense nucleotide
<400>  49
aggttcgtag tcttg     15

<210>  50
<211>  15
<212>  DNA
<213>  Artificial sequence
<220>
<223>  antisense nucleotide
<400>  50
caggttcgta gtctt     15

<210>  51
<211>  16
<212>  DNA
<213>  Artificial sequence
<220>

<223>  antisense nucleotide
<400>  51
ctccctcctt ggcctt     16

<210>  52
<211>  16
<212>  DNA
<213>  Artificial sequence
<220>
<223>  antisense nucleotide
<400>  52
caccctgttt ggtttt     16

<210>  53
<211>  16
<212>  DNA
<213>  Artificial sequence
<220>
<223>  antisense nucleotide
<400>  53
actgctgtca cacccg     16

<210>  54
<211>  16
<212>  DNA
<213>  Artificial sequence
<220>
<223>  antisense nucleotide
<400>  54
tgctccctcc actgtc     16

<210>  55
<211>  16
<212>  DNA
<213>  Artificial sequence
<220>
<223>  antisense nucleotide
<400>  55
gctgcaatgc tccctg     16

<210>  56
<211>  16
<212>  DNA
<213>  Artificial sequence
<220>
<223>  antisense nucleotide
<400>  56
ccagtggctg ctgcaa     16

<210>  57
<211>  16
<212>  DNA
<213>  Artificial sequence
<220>
<223>  antisense nucleotide
<400>  57
tgacaaagcc agtggc     16

<210>  58
<211>  16
<212>  DNA
<213>  Artificial sequence
<220>

<223>  antisense nucleotide
<400>  58
tcattgtcag gatcca    16

<210>  59
<211>  16
<212>  DNA
<213>  Artificial sequence
<220>
<223>  antisense nucleotide
<400>  59
aggttcgtag tcttga    16